# EUROPEAN PATENT APPLICATION

(11) **EP 4 796 042 A1**
(43) Date of publication of application: **26.08.2026**
(21) Application number: 25790504.2
(22) Date of filing: 08.01.2025
(51) Int. Cl.: A61B 6/00, A61B 6/50, A61B 5/00, G06T 7/00, G06N 20/20

(54) **OSTEOPOROSIS DIAGNOSIS SYSTEM AND INTERPRETATION METHOD USING X-RAY IMAGES**

(30) Priority: 19.04.2024 KR 20240052824; 26.09.2024 KR 20240130420
(60) Divisional application: 26191240.6
(71) Applicant: Promedius Inc., Seoul 05510 (KR)
(72) Inventor: JANG, Miso, Seoul 04502 (KR); LEE, Gaeun, Seongnam-si, Gyeonggi-do 13646 (KR)
(74) Representative: Lavoix
(86) International application number: PCT/KR2025/000389
(87) International publication number: WO 2025/220843

(57) **Abstract**

A system for diagnosing osteoporosis according to an embodiment includes a classification model training device that includes a plurality of classification models, one or more of the plurality of classification models being artificial intelligence models corresponding to each of a plurality of segmented images which are obtained by segmenting an entire image corresponding to each anatomical area, and trains a corresponding classification model based on the plurality of segmented images and training labels corresponding to each segmented image, the training label being labeled as normal or the osteoporosis, and a bone disease classification device that diagnoses whether there is the osteoporosis by inputting the plurality of segmented images segmented from a target image for interpretation to the corresponding classification model of the classification model training device.

## Description

### [Technical Field]

The present disclosure relates to a system and method for diagnosing osteoporosis using X-ray images, and more particularly, to a system and method for diagnosing osteoporosis using chest X-ray entire images and segmented images.

### [Background Art]

In 2018, the Republic of Korea entered an aging society with a proportion of the elderly aged 65 or older of 14%, and is expected to enter a super-aging society with a proportion of over 20% in 2025. Accordingly, the number of osteoporosis patients is expected to increase rapidly, and medical costs and socioeconomic costs due to osteoporosis are expected to increase rapidly.

The osteoporosis means that bone strength is weakened and fractures are more likely to occur, and is a skeletal disease that progresses throughout a body. The osteoporosis has no specific symptoms, but if a fracture occurs, the possibility of secondary fractures and complications increases, so it is necessary to prevent the risk of osteoporosis through screening tests for decreased bone density. The osteoporosis is diagnosed through bone density tests such as Dual-Energy X-ray Absorptiometry (hereinafter referred to as 'DEXA'), and is classified as normal, osteopenia, or osteoporosis based on the bone index (T-score) measured after the bone density test.

Recently, as image analysis technology using an artificial intelligence model has developed rapidly, the artificial intelligence model is also being used to diagnose the osteoporosis. U.S. Patent No. 12,033,318 describes a technology for estimating bone density from X-ray images using an artificial intelligence model. Specifically, according to U.S. Patent No. 12,033,318, a human skeletal image is used as training data, and bone mineral density (BMD) measured from DEXA is used as supervised data to perform training on an artificial intelligence model, and through the trained artificial intelligence model, bone density or bone indices (such as T-score) with continuity are directly predicted from chest X-ray images, and through the prediction, the bone condition or presence or absence of the bone disease may be determined.

The technology according to U.S. Patent No. 12,033,318 has the advantage of being able to provide detailed diagnostic information such as bone condition through continuous predicted values of bone density or bone indices, but in order to train a highly accurate artificial intelligence model, a large amount of medical images and paired DEXA-extracted label information (bone density or bone indices such as T-score) from various hospitals (or cohort groups) are required. Usually, the medical data deals with personal records of patients, making it difficult to access in a private sector, and since it is not standardized in a structured form suitable for training, it is not easy to secure a large amount of medical data necessary for training an Al model, and there is a problem that a lot of money is needed to secure sufficient data.

Meanwhile, X-ray images may generate unnecessary shapes or parts (hereinafter referred to as 'artifacts') during the acquisition process due to the skill of the photographer, the location or movement of the subject, differences in X-ray absorption rates, living implants, tissues other than bones, etc., and US Patent No. 12,033,318 does not describe any processing of these artifacts, so there is a problem that artifacts may be recognized as part of bone information.

Therefore, in order to learn an Al model with high accuracy from X-ray images, a procedure to detect and remove these artifacts is necessary.

### [Disclosure]

### [Technical Problem]

The present disclosure attempts to provide a system and method for diagnosing osteoporosis capable of providing accurate analysis results even in incomplete images by inferring bone-related information by combining output results from models trained on each of a plurality of extracted images using an ensemble algorithm.

In addition, the present disclosure attempts to provide a system and method for diagnosing osteoporosis capable of quickly securing training efficiency and accuracy of a training model by applying transfer learning.

### [Technical Solution]

According to an exemplary embodiment, a system for diagnosing osteoporosis includes:
a classification model training device that includes a plurality of classification models, one or more of the plurality of classification models being artificial intelligence models corresponding to each of a plurality of segmented images which are obtained by segmenting an entire image corresponding to each anatomical area, and trains a corresponding classification model based on the plurality of segmented images and training labels corresponding to each segmented image, the training label being labeled as normal or the osteoporosis; and a bone disease classification device that diagnoses whether there is the osteoporosis by inputting the plurality of segmented images segmented from a target image for interpretation to the corresponding classification model of the classification model training device.

The plurality of classification models may include a first classification model corresponding to the entire image, and the classification model training device may train a first classification model based on the entire image and the corresponding training label.

The entire image may be a chest X-ray image.

The classification model training device may include: a first image segmentation unit that segments the entire image into the anatomical areas to generate the segmented image; a training dataset generation unit that generates a plurality of training datasets based on an entire image of a normal person or an osteoporosis patient input from the outside and the plurality of segmented images segmented by the first image segmentation unit, each training dataset including a training image which is the entire image or the segmented image, and the training label labeled as the normal or the osteoporosis; and a plurality of classification model training units that train each of the corresponding classification models based on the plurality of training datasets generated by the training dataset generation unit.

The first image segmentation unit may crop and segment the entire image into images corresponding to each anatomical area, and the training image generated by the training dataset generation unit may include an entire chest image and one or more segmented images of right clavicle-scapula, left clavicle-scapula, cervical spine, and thoracic/lumbar spine.

The bone disease classification device may include: a second image segmentation unit that segments the target image for interpretation into the anatomical areas to generate the plurality of segmented images; and a bone disease classification inference unit that inputs the entire target image for interpretation and the plurality of segmented images segmented by the second image segmentation unit to the corresponding classification model among the plurality of classification models of the classification model training device, and infers bone disease classification results for each input image.

The bone disease classification device may further include a combination unit that combines a plurality of bone disease classification results inferred by the bone disease classification inference unit using an ensemble algorithm to diagnose whether the target image for interpretation for interpretation corresponds to an osteoporosis image or a normal person image.

According to another exemplary embodiment, a system for diagnosing osteoporosis includes:
a classification model training device that performs primary training on a plurality of source classification models based on first training images including segmented images segmented from entire images of a normal person and an osteoporosis patient, transfers the primary training result, and performs secondary training on a plurality of target classification models based on secondary training images including segmented images segmented from an entire images of the normal person, an osteopenia patient, and the osteoporosis patient; and a bone disease classification device that diagnoses whether there is the osteoporosis by inputting a plurality of segmented images segmented from a target image for interpretation to each of the corresponding target classification models.

The plurality of source classification models and the plurality of target classification models may each include a first source classification model and a first target classification model corresponding to the entire image.

The entire image may be a chest X-ray image.

The classification model training device may include: a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area; a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as non-osteoporosis or the osteoporosis; a plurality of source classification model training units that perform the primary training on the corresponding source classification model based on the plurality of source training datasets; and a plurality of target classification model training units that performs the secondary training on the corresponding target classification model based on the plurality of target training datasets.

The classification model training device may include: a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area; a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as normal, osteopenia, or the osteoporosis; a plurality of source classification model training units that perform the primary training on the corresponding source classification model based on the plurality of source training datasets; and a plurality of target classification model training units that performs the secondary training on the corresponding target classification model based on the plurality of target training datasets.

The classification model training device may further include a transfer learning unit that performs transfer learning on a corresponding target classification model training unit based on hidden layer parameters which are training results of the source classification model training unit.

The first image segmentation unit may crop and segment the entire image into images corresponding to each anatomical area, and the training image generated by the training dataset generation unit may include an entire chest image and one or more segmented images of right clavicle-scapula, left clavicle-scapula, cervical spine, and thoracic/lumbar spine.

The bone disease classification device may include: a second image segmentation unit that segments the target image for interpretation by anatomical areas to generate the plurality of segmented images; and a bone disease classification inference unit that inputs the entire target image for interpretation and the plurality of segmented images segmented by the second image segmentation unit to the corresponding classification model among the plurality of target classification models, and infers bone disease classification results for each input image.

The bone disease classification device may further include a combination unit that combines a plurality of bone disease classification results inferred by the bone disease classification inference unit using an ensemble algorithm to diagnose whether the target image for interpretation for interpretation corresponds to an osteoporosis image.

The bone disease classification device may diagnose the target image for interpretation as an osteoporosis image or a non-osteoporosis image.

The bone disease classification device may diagnose the target image for interpretation as an osteoporosis image, an osteopenia image, or a normal person's image.

A bone disease classification device that diagnoses whether there is the osteoporosis by accessing a classification model training device that includes a plurality of classification models corresponding to an entire image or each of a plurality of segmented images which are obtained by segmenting the entire image corresponding to each anatomical area,
in which the bone disease classification device may input a target image for interpretation or a plurality of segmented images segmented from the target image for interpretation to a corresponding classification model among the plurality of classification models to diagnose whether the target image for interpretation is the osteoporosis.

The bone disease classification device may further include: an image segmentation unit that segments the target image for interpretation into the anatomical areas to generate the plurality of segmented images; and a bone disease classification inference unit that inputs the entire target image for interpretation and the plurality of segmented images segmented by the image segmentation unit to the corresponding classification model among the plurality of classification models, and infers bone disease classification results for each input image.

The bone disease classification device may further include a combination unit that combines a plurality of bone disease classification results inferred by the bone disease classification inference unit using an ensemble algorithm to diagnose whether the target image for interpretation for interpretation corresponds to an osteoporosis image.

The bone disease classification device may diagnose the target image for interpretation as an osteoporosis image or a non-osteoporosis image.

The bone disease classification device may diagnose the target image for interpretation as an osteoporosis image, an osteopenia image, or a normal person's image.

The plurality of classification training models may be classification training models that are transferred from training results of a first classification model that is primarily trained with training data of a normal person and an osteoporosis patient and is secondarily trained with training data of a normal person, an osteopenia patient, and an osteoporosis patient.

According to an exemplary embodiment, a classification model training device may perform primary training on a plurality of source classification models based on first training images including segmented images segmented from entire images of a normal person and an osteoporosis patient, transfer the primary training result, and perform secondary training on a plurality of target classification models based on secondary training images including segmented images segmented from an entire image of the normal person, an osteopenia patient, and the osteoporosis patient.

The plurality of source classification models and the plurality of target classification models may each include a first source classification model and a first target classification model corresponding to the entire image.

The classification model training device may further include a transfer learning unit that performs transfer learning on a corresponding target classification model training unit based on hidden layer parameters which are training results of the source classification model training unit.

According to an exemplary embodiment, a method for diagnosing osteoporosis is a method for diagnosing osteoporosis that diagnoses whether there is the osteoporosis from a target image for interpretation. The method includes:
Segmenting the target image for interpretation into a plurality of images corresponding to each anatomical area; inputting the target image for interpretation and the plurality of segmented images to a corresponding classification model among a plurality of classification models to infer bone disease classification results for each input image, one or more of the plurality of classification models being artificial intelligence models corresponding to each of the plurality of segmented images which are obtained by segmenting the entire image corresponding to each anatomical area; and diagnosing whether the target image for interpretation is the osteoporosis by combining a plurality of inferred bone disease classification results using an ensemble algorithm.

The method for diagnosing osteoporosis may further include training the plurality of classification models based on the entire image of a normal person or an osteoporosis patient, in which the training of the classification models may include: segmenting the entire image into the anatomical areas to generate the segmented image; generating a plurality of training datasets based on the entire training image and the plurality of segmented images, each training dataset including a training image that is the entire image or the segmented image and a training label labeled as normal or the osteoporosis; and training corresponding classification models, respectively, based on the plurality of training datasets.

The entire image may be a chest X-ray image, and the entire image may be cropped into images corresponding to each anatomical area to generate the plurality of segmented images.

The method for diagnosing osteoporosis may further include training the plurality of classification models based on the entire image of a normal person, a osteopenia patient, or an osteoporosis patient, in which the plurality of classification training models may be a plurality of target classification training models that are transferred from training results of a plurality of source classification models that are primarily trained with a first training images of a normal person and an osteoporosis patient, and are classification training models that are secondarily trained with a second training image of a normal person, an osteopenia patient, and an osteoporosis patient.

The method for diagnosing osteoporosis may further include: segmenting a source training image, which is an entire image of the normal person or the osteoporosis patient, into images corresponding to each anatomical area; generating a plurality of source training datasets based on the source training image and a plurality of segmented images segmented from the source training image, each of the source training datasets including the first training image that is an image segmented from the source training image or the source training image, and a source training label labeled as the normal or the osteoporosis; performing the primary training on the corresponding source classification model based on the plurality of source training datasets; performing transfer learning on the corresponding target classification model based on hidden layer parameters that are training results of the source classification model; segmenting a target training image, which is the entire image of the normal person, the osteopenia patient, or the osteoporosis patient, into images corresponding to each anatomical area; generating a plurality of target training datasets based on the target training image and a plurality of segmented images segmented from the target training image, each of the target training datasets including the second training image that is an image segmented from the target training image or the target training image, and a target training label labeled as the non-osteoporosis or the osteoporosis; and performing the secondary training on the corresponding target classification model based on the plurality of target training datasets.

The method for diagnosing osteoporosis may further include: segmenting a source training image, which is an entire image of the normal person or the osteoporosis patient, into images corresponding to each anatomical area; generating a plurality of source training datasets based on the source training image and a plurality of segmented images segmented from the source training image, each of the source training datasets including the first training image that is an image segmented from the source training image or the source training image, and a source training label labeled as the normal or the osteoporosis; performing the primary training on the corresponding source classification model based on the plurality of source training datasets; performing transfer learning on the corresponding target classification model based on hidden layer parameters that are training results of the source classification model; segmenting a target training image, which is the entire image of the normal person, the osteopenia patient, or the osteoporosis patient, into images corresponding to each anatomical area; generating a plurality of target training datasets based on the target training image and a plurality of segmented images segmented from the target training image, each of the target training datasets including the second training image that is an image segmented from the target training image or the target training image, and a target training label labeled as the normal, the osteopenia, the osteoporosis; and performing the secondary training on the corresponding target classification model based on the plurality of target training datasets.

According to an exemplary embodiment, a classification model training method is a method for training a plurality of classification models based on the entire image of a normal person, an osteopenia patient, or an osteoporosis patient. The method includes:
performing primary training on a plurality of source classification models based on first training images including segmented images segmented from entire images of a normal person and an osteoporosis patient; and transferring the primary training results and performing secondary training on a plurality of target classification models based on second training images including segmented images segmented from entire images of a normal person, an osteopenia patient, and an osteoporosis patient.

The performing of the primary training may include: segmenting a source training image, which is an entire image of the normal person or the osteoporosis patient, into images corresponding to each anatomical area; generating a plurality of source training datasets based on the source training image and a plurality of segmented images segmented from the source training image, each of the source training datasets including the first training image that is an image segmented from the source training image or the source training image, and a source training label labeled as the normal or the osteoporosis; and performing the primary training on the corresponding source classification model based on the plurality of source training datasets.

The performing of the secondary training may include: performing transfer learning on the corresponding target classification model based on hidden layer parameters that are training results of the source classification model; segmenting a target training image, which is the entire image of the normal person, the osteopenia patient, or the osteoporosis patient, into images corresponding to each anatomical area; generating a plurality of target training datasets based on the target training image and a plurality of segmented images segmented from the target training image, each of the target training datasets including the second training image that is an image segmented from the target training image or the target training image, and a target training label labeled as the non-osteoporosis or the osteoporosis; and performing the secondary training on the corresponding target classification model based on the plurality of target training datasets.

The performing of the secondary training may include: performing transfer learning on the corresponding target classification model based on hidden layer parameters that are training results of the source classification model; segmenting a target training image, which is the entire image of the normal person, the osteopenia patient, or the osteoporosis patient, into images corresponding to each anatomical area; generating a plurality of target training datasets based on the target training image and a plurality of segmented images segmented from the target training image, each of the target training datasets including the second training image that is an image segmented from the target training image or the target image, and a target training label labeled as the normal, the osteopenia, the osteoporosis; and performing the secondary training on the corresponding target classification model based on the plurality of target training datasets.

A computer includes at least one processor implemented to execute a computer-readable command, in which the at least one processor may
segment a target image for interpretation into a plurality of images corresponding to each anatomical area, input the target image for interpretation and the plurality of segmented images to a corresponding classification model among a plurality of classification models to infer bone disease classification results for each input image, one or more of the plurality of classification models being artificial intelligence models corresponding to each of the plurality of segmented images which are obtained by segmenting the entire image corresponding to each anatomical area, and diagnose whether the target image for interpretation is the osteoporosis by combining the plurality of inferred bone disease classification results using an ensemble algorithm.

### [Advantageous Effects]

According to the exemplary embodiments, by combining the output results from the models trained on the plurality of extracted images using the ensemble algorithm to infer the bone-related information, it is possible to increase the prediction accuracy and provide the accurate analysis results even when the images are incomplete.

According to the exemplary embodiments, it is possible to quickly secure the training efficiency and accuracy of the training model by applying the transfer learning.

### [Brief Description of the Drawings]

FIG. 1 is a diagram illustrating a configuration of a medical imaging system according to an exemplary embodiment of the present disclosure.
FIG. 2 is a diagram illustrating a computing device implementing a system for diagnosing osteoporosis according to an exemplary embodiment of the present disclosure.
FIG. 3 is a diagram illustrating a system for diagnosing osteoporosis according to a first exemplary embodiment of the present disclosure.
FIG. 4 is a diagram illustrating a classification model training device according to the first exemplary embodiment of the present disclosure.
FIG. 5 is a diagram illustrating an example of a segmented CXR image according to an exemplary embodiment of the present disclosure.
FIG. 6 is a diagram illustrating an example of generating a plurality of training datasets according to the first exemplary embodiment of the present disclosure.
FIG. 7 is a diagram illustrating a deep neural network model according to an exemplary embodiment of the present disclosure.
FIG. 8 is a diagram illustrating a classification model training method according to the first exemplary embodiment of the present disclosure.
FIG. 9 is a diagram illustrating a bone disease classification device according to the first exemplary embodiment of the present disclosure.
FIG. 10 is a diagram illustrating a bone disease classification method according to the first exemplary embodiment of the present disclosure.
FIG. 11 is a diagram illustrating a system for diagnosing osteoporosis according to a second exemplary embodiment of the present disclosure.
FIG. 12 is a diagram illustrating a classification model training device according to a second exemplary embodiment of the present disclosure.
FIG. 13 is a diagram illustrating an example of transfer learning according to a second exemplary embodiment of the present disclosure.
FIG. 14 is a diagram illustrating a classification model training method according to a second exemplary embodiment of the present disclosure.
FIG. 15 is a diagram illustrating a bone disease classification device according to a second exemplary embodiment of the present disclosure.
FIG. 16 is a diagram illustrating the bone disease classification method according to the second exemplary embodiment of the present disclosure.
FIG. 17 is a diagram illustrating a combination process performed in a combination unit according to a second exemplary embodiment of the present disclosure.
FIG. 18 is a diagram illustrating a classification model training method according to a third exemplary embodiment of the present disclosure.
FIG. 19 is a diagram illustrating a bone disease classification method according to a third exemplary embodiment of the present disclosure.

### [Mode for Invention]

In the following detailed description, only certain embodiments of the present invention have been shown and described, simply by way of illustration. As those skilled in the art would realize, the described embodiments may be modified in various different ways, all without departing from the spirit or scope of the present invention. Accordingly, the drawings and description are to be regarded as illustrative in nature and not restrictive. Like reference numerals designate like elements throughout the specification.

Throughout the specification, unless explicitly described to the contrary, the word "comprise" and variations such as "comprises" or "comprising", will be understood to imply the inclusion of stated elements but not the exclusion of any other elements.

In addition, the terms "~unit", "~or/er", "~module", etc., described in the specification mean a unit that processes at least one function or operation, which may be implemented by hardware or software, or a combination of hardware and software.

In this specification, "transmission or provision" may include not only direct transmission or provision, but also indirect transmission or provision through another device or by using a bypass path.

In the present specification, an expression written in singular may be construed in singular or plural unless an explicit expression such as "one" or "single" is used.

In describing the exemplary embodiment of the present invention, when it is determined that a detailed description of any known art related to the present invention may obscure the gist of the present invention, the detailed description will be omitted.

Hereinafter, a preferred exemplary embodiment according to the present disclosure will be described in detail with reference to the attached drawings. In the drawings, the same reference numerals are used to indicate the same or similar components.

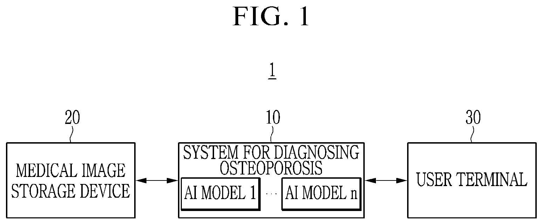

FIG. 1 is a diagram illustrating a configuration of a medical imaging system according to an exemplary embodiment of the present disclosure.

Referring to FIG. 1, a medical imaging system 1 may include at least one user terminal 30, a medical image storage device 20, and a system 10 for diagnosing osteoporosis.

The user terminal 30 is configured with hardware and software that installs programs executed by a processor and provides a computing environment and a network environment for performing the operations of the present disclosure. The user terminal 30 may be implemented in various types, such as a computing device in a workstation and a mobile device.

The user terminal 30 may be linked with the medical image storage device 20 to display medical image-related data stored in the medical image storage device 20. In addition, the user terminal 30 may display the results of bone diseases diagnosed by the system 10 for diagnosing osteoporosis. Such functions may be provided through a viewer, which is a dedicated program installed in the user terminal 30. A viewer may be installed and executed on, for example, a computing device within a workstation, and may be implemented to access the medical image storage device 20 and the system 10 for diagnosing osteoporosis, and may display medical image-related data stored in the medical image storage device 20 or bone disease information (e.g., osteoporosis) diagnosed by the system 10 for diagnosing osteoporosis.

The medical image storage device 20 may store and manage captured medical images. In addition, the medical image storage device 20 may store and manage analysis results for the medical images. The medical image storage device 20 may include a database (picture archiving and communications system (hereinafter, referred to as PACS) database) of a medical image storage and transmission system. The medical image storage device 20 may store data according to a designated data format. For example, the medical image storage device 20 may store medical images captured by medical imaging devices and analysis results of the medical images according to a digital imaging and communications in medicine (DICOM) standard, and may communicate with the user terminal 30 to provide data for image interpretation. In the exemplary embodiment of the present disclosure, the DICOM standard is used as an example of a standard used for storing the medical images, but the present disclosure is not limited thereto and may be provided in various other formats.

The medical image storage device 20 may acquire bone disease diagnosis information (e.g., osteoporosis/non-osteoporosis) from the system 10 for diagnosing osteoporosis. The medical images stored in the medical image storage device 20 may be X-ray images, magnetic resonance imaging (MRI) images, ultrasound images, computed tomography (CT) images, digital mammography (MMG) images, digital breast tomosynthesis (DBT) images, etc.

In the exemplary embodiment of the present disclosure, a chest X-ray image (hereinafter also simply referred to as an 'X-ray image') is described as an example of a medical image, but the present disclosure need not be limited thereto, and the present disclosure may be applied to a type of medical images.

The system 10 for diagnosing osteoporosis analyzes a chest X-ray (hereinafter also referred to as 'CXR') image using a plurality of artificial intelligence (AI) models, and diagnoses whether there is a bone disease (e.g., whether there is osteoporosis) based on the CXR analysis results.

The system 10 for diagnosing osteoporosis according to the exemplary embodiment of the present disclosure may be equipped with a plurality of artificial intelligence models (hereinafter also referred to as 'learning models' or 'classification models') specialized for each of the entire CXR image and a plurality of segmented CXR images, and after dividing the input entire CXR image into the plurality of segmented CXR images by reflecting anatomical features, the entire image and each segmented image may be input to a corresponding artificial intelligence model to determine whether there is the bone disease (whether there is osteoporosis). The artificial intelligence model (AI model) according to the exemplary embodiment of the present disclosure is generated to make medical inferences from input medical images, and a model structure, a training data configuration, a training method, medical inference targets, etc., may be designed in various ways.

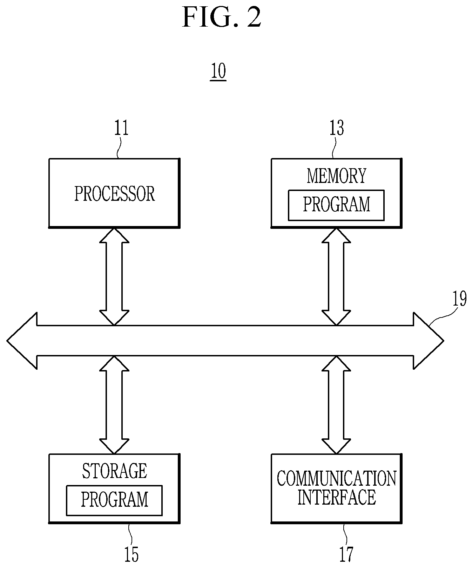

The system 10 for diagnosing osteoporosis or the detailed device within the system for diagnosing osteoporosis according to the exemplary embodiment of the present disclosure may be implemented as a computing device as illustrated in FIG. 2.

Referring to FIG. 2, the system for diagnosing osteoporosis or the detailed device (computing device, 10) within the system for diagnosing osteoporosis may include one or more processors 11, a memory 13 for loading a program executed by the processor 11, a storage 15 for storing the program and various data, a communication interface 17, and a bus 19 connecting them. In addition, the system 10 for diagnosing osteoporosis may further include various components.

The program may include instructions that cause the processor 11 to perform methods/operations according to various exemplary embodiments of the present disclosure when loaded into the memory 13. That is, the processor 11 may perform methods/operations according to various exemplary embodiments of the present disclosure by executing the instructions. The program is composed of a series of computer-readable instructions grouped based on function, and refers to something that is executed by the processor.

The processor 11 controls the overall operation of each component of the computing device 10. The processor 11 may be configured as a single physical entity, but may also be configured as the plurality of entities. The processor 11 configured as the plurality of entities may process a single execution element by dividing the single execution element or may process a plurality of execution elements by dividing the plurality of execution elements.

The processor 11 may be configured to include at least one of a central processing unit (CPU), a microprocessor unit (MPU), a micro controller unit (MCU), a graphic processing unit (GPU), or any type of processor well known in the art of the present invention. In addition, the processor 11 may perform operations on at least one application or program to execute the methods/operations according to various exemplary embodiments of the present disclosure.

The memory 13 stores various data, instructions and/or information. The memory 13 may load one or more programs from the storage 15 to execute the methods/operations according to various exemplary embodiments of the present disclosure. The memory 13 may be implemented as a volatile memory such as RAM, but the technical scope of the present disclosure is not limited thereto.

The storage 15 may non-temporarily store the programs. The storage 15 may be configured to include a nonvolatile memory, such as a read only memory (ROM), an erasable programmable ROM (EPROM), an electrically erasable programmable ROM (EEPROM), and a flash memory, a hard disk, a removable disk, or any well-known computer-readable recording medium in the art to which the present disclosure belongs.

According to an exemplary embodiment of the present disclosure, the storage may store a plurality of classification models and trained parameters of each classification model. In this case, each classification model may be a plurality of artificial intelligence models (training models) specialized for the entire X-ray image or a plurality of segmented X-ray images, and may be an artificial intelligence model having a neural network structure that trains the relationship between an input image and a label.

The communication interface 17 supports wired and wireless Internet communication of the computing device 10. In addition, the communication interface 17 may support various communication methods other than the Internet communication. To this end, the communication interface 17 may be configured to include a communication module well known in the technical field of the present disclosure.

The bus 19 provides a communication function between components of the computing device 10. The bus 19 may be implemented as various types of buses, such as an address bus, a data bus, and a control bus.

The computer program includes instructions executed by the processor 11 and stored on a non-transitory computer readable storage medium. The instructions cause the processor 11 to execute the operation according to an exemplary embodiment. The computer program may be downloaded through the network or sold in the product form.

The computer program according to the exemplary embodiment of the present disclosure may include commands for collecting a dataset for pre-training of the artificial intelligence model and training the artificial intelligence model using the collected dataset. The computer program may include instructions for preprocessing input data and training the artificial intelligence model using the preprocessed input data.

The functional blocks (indicated as "~units") of the detailed devices of the system 10 for diagnosing osteoporosis described below may be implemented by cooperation of programs, etc., included in the processor 11 and the memory 13 (or the storage 15), which are detailed components of the computing device illustrated in FIG. 2.

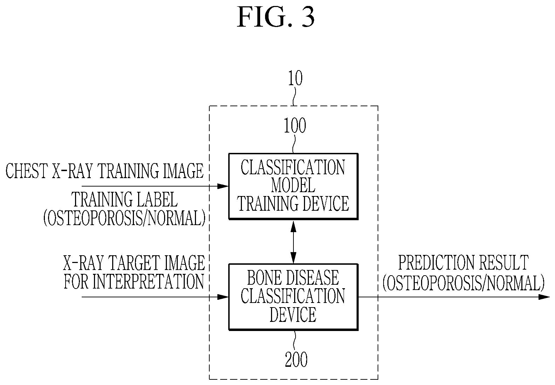

Hereinafter, the system 10 for diagnosing osteoporosis according to the first exemplary embodiment of the present disclosure will be described with reference to FIGS. 3 to 10.

Referring to FIG. 3, the system 10 for diagnosing osteoporosis according to the first exemplary embodiment of the present disclosure may include a classification model training device 100 and a bone disease classification device 200.

The classification model training device 100 includes the plurality of classification models. Each classification model may be the artificial intelligence model corresponding to the entire CXR image (hereinafter also referred to simply as an 'entire image') or a plurality of segmented CXR images (hereinafter also referred to simply as 'segmented images') that are segmented into each anatomical area (e.g., entire chest, left clavicle-scapula, right clavicle-scapula, cervical spine, and thoracic/lumbar spine) of the entire CXR image.

According to the first exemplary embodiment of the present disclosure, the classification model training device 100 receives an entire CXR image (entire image) of a normal person or an osteoporosis patient as a training image. That is, the classification model training device 100 receives the entire image of a normal person or an osteoporosis patient and the corresponding training label (osteoporosis/normal) as the training data.

As described below, the classification model training device 100 segments the input entire image into a plurality of segmented images corresponding to the anatomical areas, generates a training dataset based on the entire image or the segmented image and the input training label (osteoporosis/normal), and then trains the corresponding classification model using each training dataset.

The classification model (artificial intelligence model) used in the exemplary embodiment of the present disclosure may include a deep neural network structure. The deep neural network may be a structure including a plurality of hidden layers between an input layer and an output layer. Deep neural network algorithms such as a deep neural network (DNN), a convolutional neural network (CNN), a recurrent neural network (RNN), a restricted Boltzmann machine (RBM), a deep belief network (DBM), a Q network, a long short-term memory (LSTM), a gated recurrent unit (GRU), a transformer, a capsule network, etc., may be used, and the present disclosure is not limited thereto, and other artificial intelligence models may be used.

According to an exemplary embodiment of the present disclosure, the classification model training device 100 uses the plurality of classification models having the same neural network structure. However, the present disclosure is not limited thereto, and the classification models having different neural network structures may be used depending on the characteristics of the entire image or the segmented image.

The bone disease classification device 200 receives a CXR image to be read (hereinafter also referred to as a 'target image') and inputs the received target image into the plurality of classification models that have completed learning performed by the classification model training device 100 to predict whether there is the bone disease (whether there is osteoporosis). Specifically, as described below, the bone disease classification device 200 segments the input target image into the plurality of segmented images corresponding to the anatomical areas, inputs the plurality of classification models that have completed training corresponding to the entire image or the segmented image, and infers the bone disease classification for each of the plurality of models. Then, the bone disease classification device 200 diagnoses whether there is a final bone disease using an ensemble algorithm for a plurality of inferred bone disease classification results.

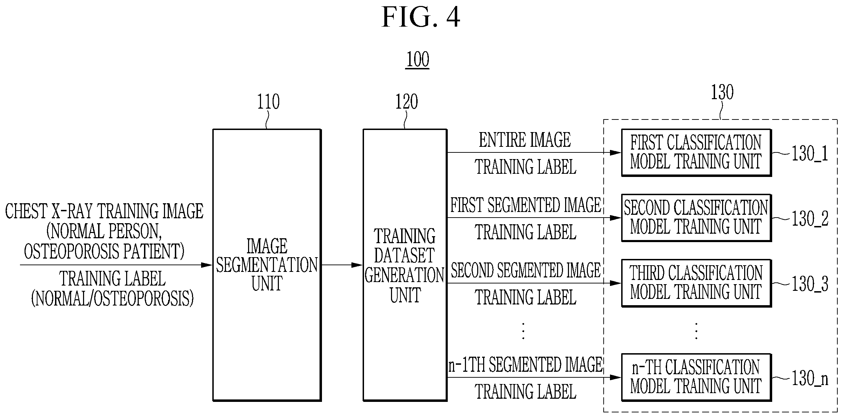

Referring to FIG. 4, the classification model training device 100 according to the first exemplary embodiment of the present disclosure may include an image segmentation unit 110, a training dataset generation unit 120, and a plurality of classification model training units 130.

The image segmentation unit 110 receives an entire CXR image of a normal or osteoporosis patient as the training target and the training label (normal/osteoporosis) information related thereto, and segments the entire CXR image into images corresponding to each of the anatomical areas.

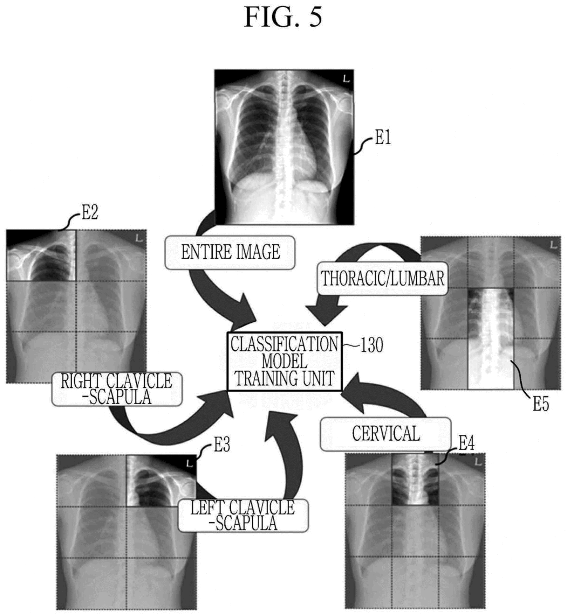

FIG. 5 is a diagram illustrating an example of a CXR image segmented by the image segmentation unit 110 and the entire CXR image according to an exemplary embodiment of the present disclosure.

Referring to FIG. 5, the image segmentation unit 110 according to the exemplary embodiment of the present disclosure crops the entire CXR image by the anatomical area to generate the segmented CXR images. Specifically, the image segmentation unit 110 may crop the entire CXR image by the anatomical area to generate the segmented images corresponding to an entire chest image E1, a right clavicle-scapula E2, a left clavicle-scapula E3, a cervical spine E4, and a thoracic/lumbar spine E5. In this way, according to the exemplary embodiment of the present disclosure, since the original CXR image is divided into equal parts and each of the anatomical areas is used as a training image without using a separate artificial intelligence model that detects bone areas or performs segmentation in the entire CXR image, there is an advantage of saving a preprocessing time and computing resources of data used in the classification model.

However, the present disclosure is not limited to the method of segmenting an entire image into images corresponding to each of the anatomical areas and cropping the images, and various image segmentation techniques such as the use of the segmentation model may be used in the present disclosure.

In addition, in the exemplary embodiment of the present disclosure, the entire chest image E1, the right clavicle-scapula E2, the left clavicle-scapula E3, the cervical spine E4, and the thoracic/lumbar spine E5 were used as the training images, but the present disclosure is not limited thereto, and the entire CXR image and images (for example, the right clavicle-scapula image and the left clavicle-scapula image) corresponding to some of the anatomical areas may be used, or only the segmented images may be used instead of the entire image.

The training dataset generation unit 120 generates the plurality of training datasets based on the entire CXR image input from the outside, the training label (normal/osteoporosis) related thereto, and the plurality of segmented images segmented by the image segmentation unit 110.

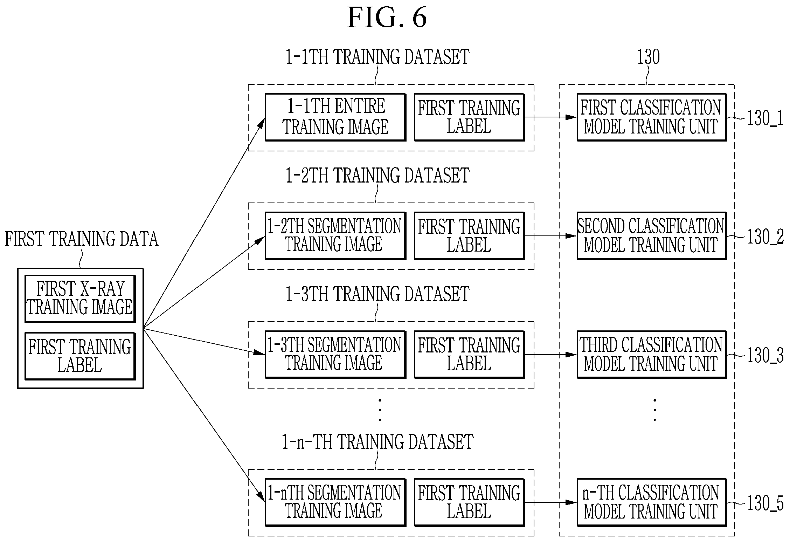

FIG. 6 is a diagram illustrating an example of generating a plurality of training datasets according to the first exemplary embodiment of the present disclosure.

Referring to FIG. 6 of FIG. 103, the training dataset generation unit 120 generates the plurality of training datasets (1-1-th training dataset, 1-2-th training dataset, 1-3-th training dataset, ..., 1-n-th training dataset) based on a first X-Ray training image and a first training label, which are the input first training data, and the plurality of segmented images segmented by the image segmentation unit 110. Specifically, the training dataset generation unit 120 generates the 1-1-th training dataset based on the input first X-Ray training image (1-1-th entire training image) and the first training label corresponding thereto, generates a 1-2-th training dataset based on the 1-2-th segmented training image (e.g., right clavicle-scapula image) segmented by the image segmentation unit 110 and the first training label (label corresponding to the entire image), and generates a 1-3-th training dataset based on the 1-3-th segmented training image (e.g., left clavicle-scapula image) segmented by the image segmentation unit 110 and the first training label. In the same manner, a 1-n-the training dataset may be generated based on the 1-n-th segmented training image segmented by the image segmentation unit 110 and the first training label.

Referring to FIG. 6, it may be seen that the training labels included in the plurality of training datasets generated by the training dataset generation unit 120 are all generated identically to the training labels (first training label) corresponding to the input CXR images (first X-Ray training image).

The plurality of training datasets generated by the training dataset generation unit 120 are used as the training data for the corresponding classification models, respectively. For example, the 1-1-th training dataset set, the 1-2-th training dataset set, and the 1-3-th training dataset set are used as training data for a first classification model, a second classification model, and a third classification model, respectively.

The classification model training unit 130 may include a plurality of classification model training units 130-1, 130-2, 130-3, ..., and 130_n that train the plurality of training datasets, respectively. For example, the first classification model training unit 130_1 trains the first classification model (artificial intelligence model) using a 1-1-th entire training image and the first training label, which are a 1-1-th dataset generated by the training dataset generation unit 120. The second classification model training unit 130_2 trains the second classification model (artificial intelligence model) using a 1-2-th segmented training image and the first training label, which are the 1-2-th dataset generated by the training dataset generation unit 120. Similarly, the n-th classification model training unit 130_n trains the n-th classification model (artificial intelligence model) using the 1-n-th segmented training image and the first training label, which are a 1-n-th dataset generated by the training dataset generation unit 120.

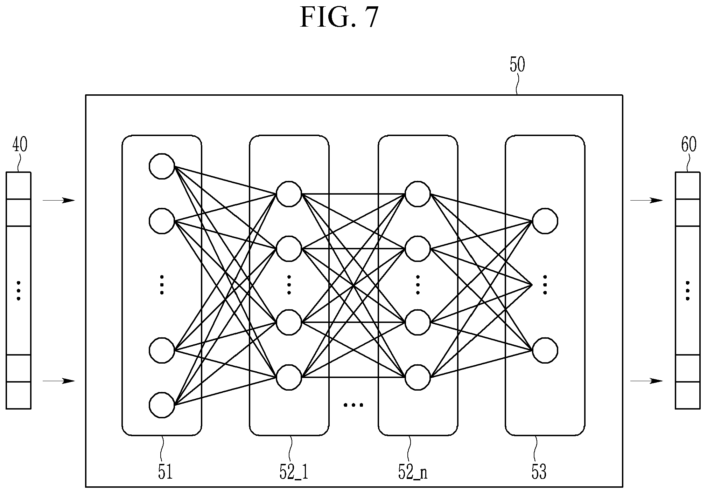

FIG. 7 is an exemplary diagram illustrating a deep neural network model 50 used in a classification model according to an exemplary embodiment of the present disclosure. The neural network model 50 is an example of a machine learning model, and in machine learning technology and cognitive science, it is a statistical learning algorithm implemented based on a structure of a biological neural network or a structure that executes the algorithm.

According to an exemplary embodiment, the deep neural network model 50 may represent a machine learning model having problem-solving capabilities that allows nodes, which are artificial neurons that form a network by combining synapses like in a biological neural network, to iteratively adjust weights of synapses to perform learning so that errors between a correct output corresponding to a specific input and an inferred output are reduced. For example, the neural network model 50 may include any probability model, neural network model, etc., that are used in artificial intelligence learning methods such as machine learning and deep learning.

The deep neural network model 50 is implemented as a multilayer perceptron (MLP) composed of multilayer layers of nodes and connections between the nodes. The deep neural network model 50 according to an exemplary embodiment may be implemented using one of various artificial neural network model structures including the MLP.

As illustrated in FIG. 7, the neural network model 50 is composed of an input layer 51 that receives an input signal or data 40 from the outside, an output layer 53 that outputs an output signal or data 60 corresponding to the input data, and n hidden layers 52_1 to 52_n that are located between the input layer 51 and the output layer 53 and receive a signal from the input layer 51 to extract characteristics and transmit the extracted characteristics to the output layer 53 (where n is a positive integer). Here, the output layer 53 receives signals from the hidden layers 52_1 to 52_n and outputs the received signals to the outside.

In this way, a plurality of input variables and a plurality of output variables corresponding thereto are respectively matched in the input layer 51 and the output layer 53 of the neural network model 50, and the synapse values (weights) between the nodes included in the input layer 51, the hidden layers 52_1 to 52_n, and the output layer 53 are adjusted, so learning may be performed so that the correct output corresponding to the specific input may be extracted. By this learning process, the characteristics hidden in the input variables of the neural network model 50 may be identified, and the synapse values (or weights) between the nodes of the neural network model 50 may be adjusted so that errors between the output variables calculated based on the input variables and the target output are reduced.

According to an exemplary embodiment of the present disclosure, each classification model training unit 130 inputs the input training image (entire training image or segmented image) corresponding to the input layer 51 and inputs the training labels (normal/osteoporosis) through the output layer 53, thereby training the corresponding classification model. That is, each classification model training unit 130 performs learning based on the input training image (entire training image or segmented image) and the training labels (normal/osteoporosis), and may adjust the weights indicating the connection strength between a specific node of a current layer and a node of a previous layer.

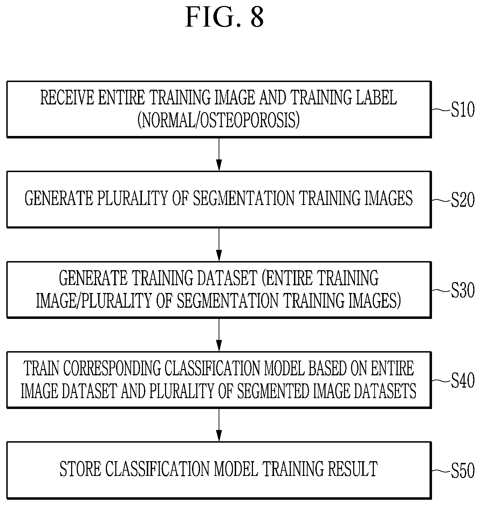

The following describes a classification model training method according to the first exemplary embodiment of the present disclosure with reference to FIG. 8. FIG. 8 is a diagram illustrating a classification model training method according to the first exemplary embodiment of the present disclosure.

The classification model training device 100 receives the entire CXR image (entire image) of the normal person or the osteoporosis patient as the training image. That is, the classification model training device 100 receives the entire image of a normal person or an osteoporosis patient and the corresponding training labels (osteoporosis/normal) as the training data. (S10)

Thereafter, the classification model training device 100 segments the input entire image into the plurality of segmented images corresponding to the anatomical areas. (S20)

According to an exemplary embodiment of the present disclosure, the image segmentation unit 110 crops the input entire CXR image by the anatomical areas to generate the segmented CXR images. Specifically, the image segmentation unit 110 may crop the entire CXR image by the anatomical area to generate the segmented images corresponding to the entire chest image E1, the right clavicle-scapula E2, the left clavicle-scapula E3, the cervical spine E4, and the thoracic/lumbar spine E5.

The training dataset generation unit 120 generates the plurality of training datasets based on the entire image or the segmented image and the input training labels (osteoporosis/normal) related to the entire image. (S30)

The classification model training unit 130 uses the plurality of training datasets generated by the training dataset generation unit 120 as the training data for the corresponding classification model, respectively, and trains the corresponding classification model. (S40)

The classification model training unit 130 stores the learning parameters (weights, biases, etc.) as the classification model training results based on the training dataset in memory or storage. (S50)

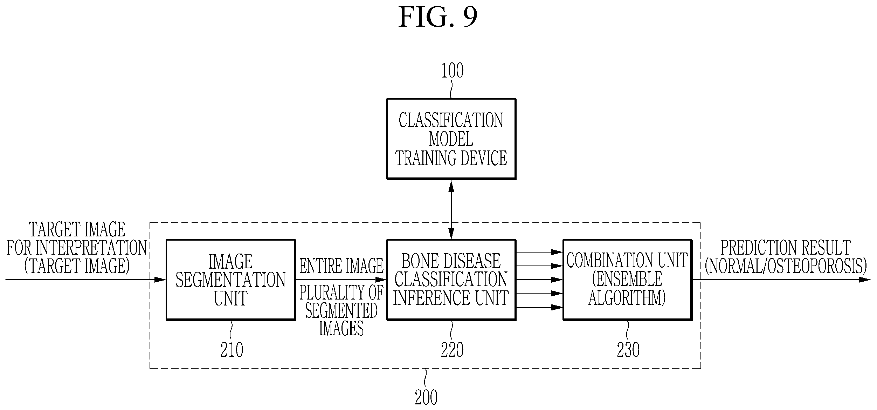

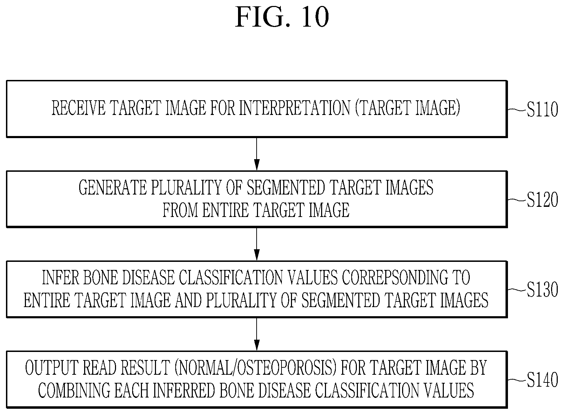

The following describes the bone disease classification device and the bone disease classification method according to the first exemplary embodiment of the present disclosure with reference to FIGS. 9 and 10.

Referring to FIG. 9, the bone disease classification device 200 according to the first exemplary embodiment of the present disclosure may include an image segmentation unit 210, a bone disease classification inference unit 220, and a combination unit 230.

The image segmentation unit 210 receives the CXR image to be read (hereinafter also referred to as the 'target image') (S110), and segments the entire target image into images corresponding to each of the anatomical areas. (S120)

In this case, the image segmentation unit 210 of the bone disease classification device 200 segments the entire image into the images corresponding to each of the anatomical areas using the same segmentation method as the image segmentation unit 210 of the classification model training device 100. According to an exemplary embodiment of the present disclosure, the image segmentation unit 210 applies a simple method of cropping the entire CXR image by the anatomical area to generate the segmented CXR images, but the present disclosure is not limited thereto, and a separate artificial intelligence model that performs segmentation may be used for segmentation.

The bone disease classification inference unit 220 inputs the entire target image and the plurality of segmented images segmented by the image segmentation unit 210 into the corresponding classification model among the plurality of trained classification models performed by the classification model training device 100, and infers the bone disease classification results for each image (entire image or segmented image). (S130)

In this case, the classification results inferred by the bone disease classification inference unit 220 may be logit or probability values.

The combination unit 230 combines the plurality of bone disease classification results inferred by the bone disease classification inference unit 220 using the ensemble algorithm to diagnose whether there is a final bone disease (normal or osteoporosis). (S140)

In this case, the ensemble algorithm according to the exemplary embodiment of the present disclosure may combine (synthesize) the plurality of bone disease classification results using an average technique. For example, the combination unit 230 may average the logit or probability values, which are the bone disease classification result values inferred from each classification model, and compare the average value with a reference value to determine whether there is the bone disease (normal/osteoporosis). In this case, according to the exemplary embodiment of the present disclosure, when combining the plurality of bone disease classification results, the combination unit 230 may exclude one or more results and calculate the average value only for the remaining results to determine whether there is the bone disease (normal/osteoporosis). For example, the logit or probability values, which are the bone disease classification result values inferred from each classification model, the logit or probability value having the maximum or minimum value are excluded, and only the remaining classification result values are averaged to determine whether there is the bone disease (normal/osteoporosis).

Meanwhile, according to an exemplary embodiment of the present disclosure, various techniques other than averaging may be applied as an ensemble technique. For example, one or a combination of voting, bagging, boosting, and stacking may be used as an ensemble technique.

According to the first exemplary embodiment of the present disclosure described above, the classification model training device 100 uses osteoporosis or normal CXR images as training input images, and the bone disease classification device 200 diagnoses the osteoporosis or normal as the final bone disease classification results for the target image. In this way, since training is performed using only the osteoporosis and normal images (i.e., images with a clear difference), the learning may be performed relatively easily even with a small amount of training data.

However, the bone disease classification may include osteopenia in addition to the osteoporosis and normal, as shown in Table 1 below. The bone disease classification may be categorized based on a T-score measured from the DEXA.

**(Table 1)**

| Bone disease classification | T-Score |
|---|---|
| Normal | -1.0 or more |
| Osteopenia | -2.5 to -1.0 |
| Osteoporosis | Less than -2.5 |

In order to train the classification model (artificial intelligence model) using data (image data) of osteopenia patients in addition to data of normal and osteoporosis patients as training data, a large amount of training data is required, but it is not easy to secure a large amount of medical data required for training the artificial intelligence model, and there is a problem that a lot of cost is required to secure sufficient data.

Accordingly, in a second exemplary embodiment of the present disclosure, a system for diagnosing osteoporosis is disclosed, which performs primary training on the first classification model (hereinafter also referred to as a 'source classification model') with training data of normal person and osteoporosis patients, which are relatively easy to train, and transfers the results of the primary training and performs secondary training on the second classification model (hereinafter also referred to as a 'target classification model') with training data of the normal person, the osteopenia patient, and the osteoporosis patient, which are difficult to train.

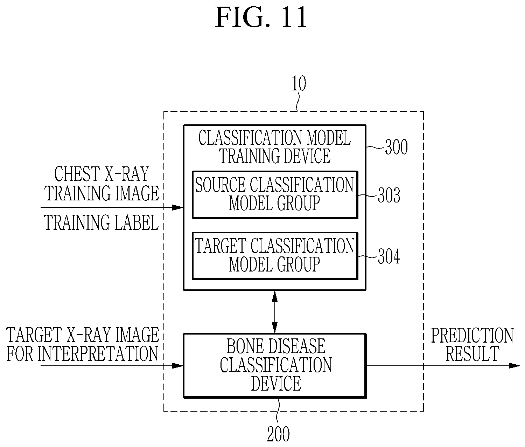

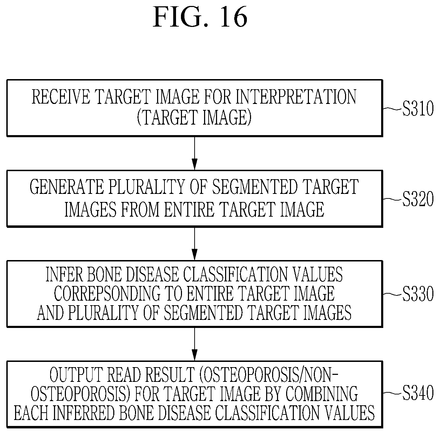

Hereinafter, a system 10 for diagnosing osteoporosis according to the second exemplary embodiment of the present disclosure will be described with reference to FIGS. 11 to 17. Hereinafter, descriptions of overlapping parts with the first exemplary embodiment of the present disclosure will be omitted, and differences will be mainly described.

Referring to FIG. 11, the system 10 for diagnosing osteoporosis according to the second exemplary embodiment of the present disclosure may include a classification model training device 300 and a bone disease classification device 200.

The classification model training device 300 may include a source classification model group 303 composed of a plurality of source classification models (artificial intelligence models) and a target classification model group 304 composed of a plurality of target classification models.

Each source classification model and each target classification model may be the artificial intelligence models corresponding to the entire CXR image or the plurality of segmented CXR images that are segmented into each of the anatomical areas of the entire CXR image.

According to the second exemplary embodiment of the present disclosure, the classification model training device 300 receives the entire CXR image (entire image) of the normal person, the osteopenia patient, or the osteoporosis patient as the training image.

As described below, the classification model training device 300 trains the source classification model group 303 based on the training image and the training labels (normal/osteoporosis) of the normal person and the osteoporosis patient, which are relatively easy to train, and transfers the hidden layer parameters, which are the training results of the source classification model group 303, to the target classification model group 304, and then trains the target classification model group 304 based on the training image and the training labels (non-osteoporosis/osteoporosis) of the normal person, the osteopenia patient, and the osteoporosis patient, which are difficult to train.

In this way, according to the second exemplary embodiment of the present disclosure, the classification model training device 300 receives the entire CXR image (entire image) of the normal person or the osteoporosis patient and the corresponding training labels (normal/osteoporosis) as source training data for training the source classification model group 303. In addition, the classification model training device 300 receives the entire CXR image (entire image) of the normal person, the osteopenia patient, or the osteoporosis patient and the corresponding training labels (non-osteoporosis/osteoporosis) as target training data for training the target classification model group 304. That is, the classification model training device 300 according to the second exemplary embodiment of the present disclosure receives the training labels (non-osteoporosis/osteoporosis) divided into two categories for the entire CXR image of the normal person, the osteopenia patient, or the osteoporosis patient as the target training label for training the target classification model group 304. Here, the target training labels of the "non-osteoporosis" is the training label corresponding to the normal person and osteopenia images as a classification other than the osteoporosis, and the target training label of the "osteoporosis" is the training label corresponding to the osteoporosis image.

According to the second exemplary embodiment of the present disclosure, since the target classification model group 304 is trained through the target training labels (non-osteoporosis/osteoporosis) divided into two categories, there is an advantage in that the target classification model group 304 may be sufficiently trained even with a relatively small number of images of the normal person, the osteopenia patient, and the osteoporosis patient.

The source classification model and the target classification model used in the second exemplary embodiment of the present disclosure may include a deep neural network structure. Deep neural network algorithms such as a deep neural network (DNN), a convolutional neural network (CNN), a recurrent neural network (RNN), a restricted Boltzmann machine (RBM), a deep belief network (DBM), a Q network, a long short-term memory (LSTM), a gated recurrent unit (GRU), a transformer, a capsule network, etc., may be used, and the present disclosure is not limited thereto, and other artificial intelligence models may be used.

In this case, according to the second exemplary embodiment of the present disclosure, the plurality of source classification models and target classification models included in each of the source classification model group and the target classification model may use the artificial intelligence models having the same neural network structure. However, the present disclosure is not limited thereto, and the source classification model group and the target classification model group may use classification models having different neural network structures.

The bone disease classification device 200 may receive the CXR image to be read (hereinafter also referred to as a 'target image') and input the received target image to the trained target classification model group 304 performed by the classification model training device 300 to predict whether there is the bone disease (whether there is the osteoporosis or non-osteoporosis). Specifically, the bone disease classification device 200 segments the input target image into the plurality of segmented images corresponding to the anatomical areas, inputs each of the entire image or the segmented image to the corresponding trained target classification models, and infers the bone disease classification for each of the plurality of target classification models. Then, the bone disease classification device 200 diagnoses whether there is the final bone disease using the ensemble algorithm for the plurality of inferred bone disease classification results.

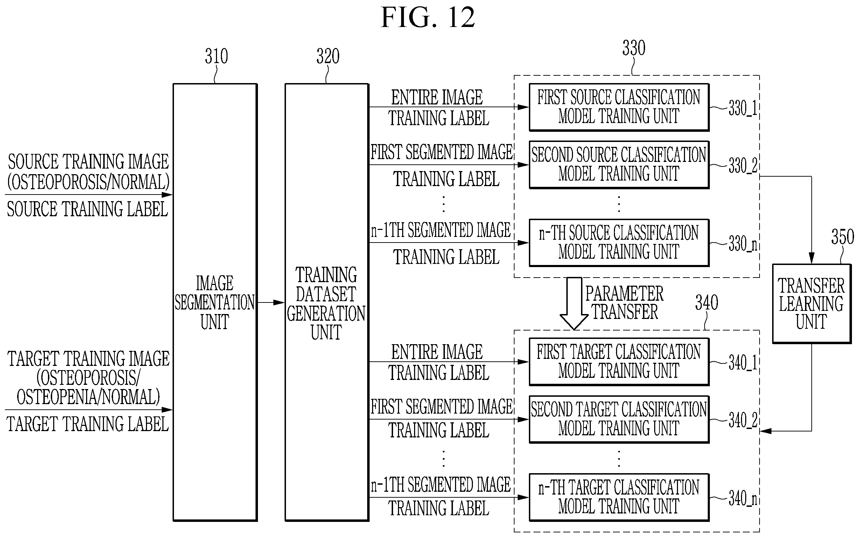

Referring to FIG. 12, the classification model training device 300 according to the second exemplary embodiment of the present disclosure may include an image segmentation unit 310, a training dataset generation unit 320, a plurality of source classification model training units 330, a plurality of target classification model training units 340, and a transfer learning unit 350.

The image segmentation unit 310 receives a source training image, which is a full CXR image of the normal person and the osteoporosis patient, and a source training label (normal/osteoporosis), and segments the source training image into images corresponding to each of the anatomical areas. In addition, the image segmentation unit 310 receives a target training image, which is a full CXR image of a normal person, an osteopenia patient, and an osteoporosis patient, and the target training label (non-osteoporosis/osteoporosis) corresponding thereto, and segments the target training image into images corresponding to each of the anatomical areas.

The training dataset generation unit 320 generates the plurality of source training datasets based on the entire CXR image input as the source training images and the source training labels (normal/osteoporosis) related thereto, and the plurality of segmented images segmented by the image segmentation unit 310. In addition, the training dataset generation unit 320 generates the plurality of target training datasets based on the entire CXR image input as a target training image and the target training labels (non-osteoporosis/osteoporosis) related thereto, and the plurality of segmented images segmented by the image segmentation unit 310.

The source classification model training unit 330 trains the source classification model group 303 based on the source training images and source training labels (normal/osteoporosis) of the normal person and the osteoporosis patient, which are relatively easy to train. The source classification model training unit 330 includes a plurality of source classification model training units 330-1, 330-2, 330-3,., and 330_n that each train a plurality of source training datasets. For example, the first source classification model training unit 330_1 trains a first source classification model (artificial intelligence model) using the entire source training image and source training label generated by the training dataset generation unit 320. The second source classification model training unit 330_2 trains the second source classification model (artificial intelligence model) using the first segmented image and source training label generated by the training dataset generation unit 320.

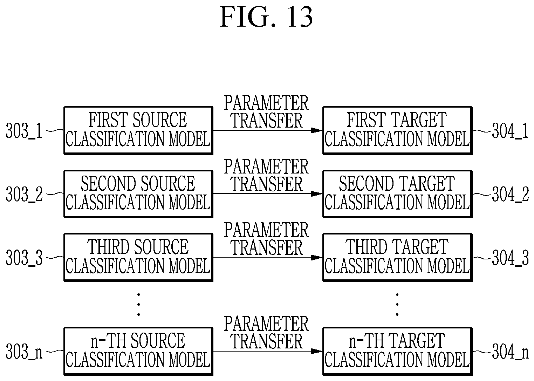

The transfer learning unit 350 transfers the hidden layer parameters, which are the training results of the source classification model group 303, to the target classification model group 304. FIG. 13 is a diagram illustrating that the transfer learning unit 350 transfers the hidden layer parameters, which are the training results of the source classification model group 303, to the target classification model group 304.

Referring to FIG. 13, the transfer learning unit 350 transfers and sets at least some of the parameters of the source classification model to the parameters of the corresponding target classification model. Specifically, the transfer learning unit 350 transfers and sets at least some of the parameters of the first source classification model to the parameters of the first target classification model, and transfers and sets at least some of the parameters of the second source classification model to the parameters of the second target classification model. In the same manner, the transfer learning unit 350 transfers and sets at least some of parameters of an n-th source classification model to parameters of an n-th target classification model.

The target classification model training unit 340 trains the target classification model group 304 in which the hidden layer parameters, which are the training results of the source classification model group 303, are transfer-learned based on the target training images and the target training labels (non-osteoporosis/osteoporosis) of the normal person, the osteopenia patient, and the osteoporosis patient.

The target classification model training unit 340 includes a plurality of target classification model training units 340-1, 340-2, 340-3, ..., and 340_n that each train the plurality of target training datasets. For example, the first target classification model training unit 340_1 trains the first target classification model (artificial intelligence model) using the entire target training image and target training label generated by the training dataset generation unit 320. The second target classification model training unit 340_2 trains the second target classification model (artificial intelligence model) using the first segmented image and target training label generated by the training dataset generation unit 320.

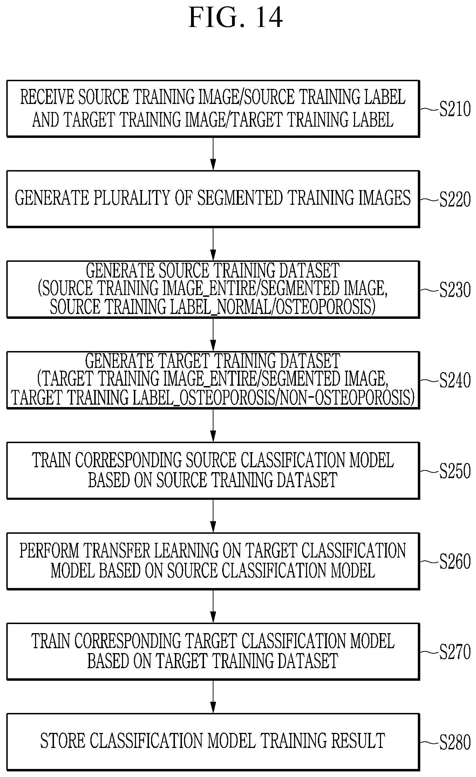

The following describes a classification model training method according to the second exemplary embodiment of the present disclosure with reference to FIG. 14. FIG. 14 is a diagram illustrating a classification model training method according to the second exemplary embodiment of the present disclosure.

The classification model training device 300 receives the source training images/source training labels and the target training images/target training labels as the training data. (S210)

That is, the classification model training device 300 receives the source training images, which are the full CXR images of the normal person and the osteoporosis patient, and the corresponding source training labels (normal/osteoporosis), and receives the target training images, which are the full CXR images of the normal person, the osteopenia patient, and the osteoporosis patient, and the corresponding target training labels (non-osteoporosis/osteoporosis).

Thereafter, the image segmentation unit 310 of the classification model training device 300 segments the received source training image and target training image into the plurality of segmented images each corresponding to the anatomical areas. (S220)

According to an exemplary embodiment of the present disclosure, the image segmentation unit 310 crops the entire CXR image input by the anatomical areas to generate the segmented CXR images. Specifically, the image segmentation unit 310 crops the entire CXR image by the anatomical area to generate the segmented images corresponding to the entire chest image, right clavicle-scapula, left clavicle-scapula, cervical spine, and thoracic/lumbar spine.

The training dataset generation unit 320 generates the plurality of source training datasets based on the entire CXR image input as the source training images and the source training labels (normal/osteoporosis) corresponding thereto, and the plurality of segmented images segmented by the image segmentation unit 310. (S230)

The training dataset generation unit 320 generates the plurality of target training datasets based on the entire CXR image input as a target training image and the target training labels (non-osteoporosis/osteoporosis) corresponding thereto, and the plurality of segmented images segmented by the image segmentation unit 310. (S240)

The source classification model training unit 330 trains the corresponding source classification model by using the plurality of source training datasets composed of the training images of the normal person and the osteoporosis patient, which is relatively easy to train, and the source training labels (normal/osteoporosis), respectively, as the training data for the corresponding source classification model. (S250)

The transfer learning unit 350 performs the transfer learning on the target classification model based on the trained source classification model. (S260)

That is, the transfer learning unit 350 performs the transfer learning on the corresponding target classification model based on the artificial intelligence neural network parameters of the trained source classification model. (S260)

The target classification model training unit 340 uses the plurality of target training datasets composed of the target training images and the target training labels (non-osteoporosis/osteoporosis) of the normal person, the osteopenia patient, and the osteoporosis patient for the transfer-learned target classification model as the training data for the corresponding target classification model, and trains the corresponding target classification model. (S270)

### (S270)

The target classification model training unit 340 stores the learning parameters (weights, biases, etc.) which are the training results of the target classification model based on the target training dataset in memory or storage.

### (S280)

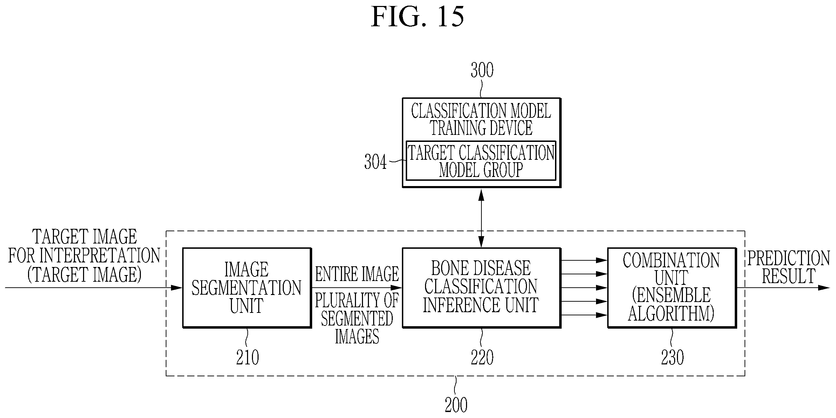

The following describes the bone disease classification device and the bone disease classification method according to the second exemplary embodiment of the present disclosure with reference to FIGS. 15 to 17.

Referring to FIG. 15, the bone disease classification device 200 according to the second exemplary embodiment of the present disclosure may include the image segmentation unit 210, the bone disease classification inference unit 220, and the combination unit 230.

The image segmentation unit 210 receives the CXR image to be read (hereinafter also referred to as the 'target image') (S310), and segments the entire target image into images corresponding to each of the anatomical areas. (S320)

In this case, the image segmentation unit 210 of the bone disease classification device 200 segments the entire image into the images corresponding to each of the anatomical areas using the same segmentation method as the image segmentation unit 210 of the classification model training device 300.

The bone disease classification inference unit 220 inputs the entire target image and the plurality of segmented images segmented by the image segmentation unit 210 into the corresponding classification model among the plurality of trained target classification model groups 304 performed by the classification model training device 300, and infers the bone disease classification results for each image (entire image or segmented image). (S330)

In this case, the classification results inferred by the bone disease classification inference unit 220 may be the logit or probability values.

The combination unit 230 combines the plurality of bone disease classification results inferred by the bone disease classification inference unit 220 using the ensemble algorithm to diagnose whether there is the final bone disease (osteoporosis/non-osteoporosis). (S340)

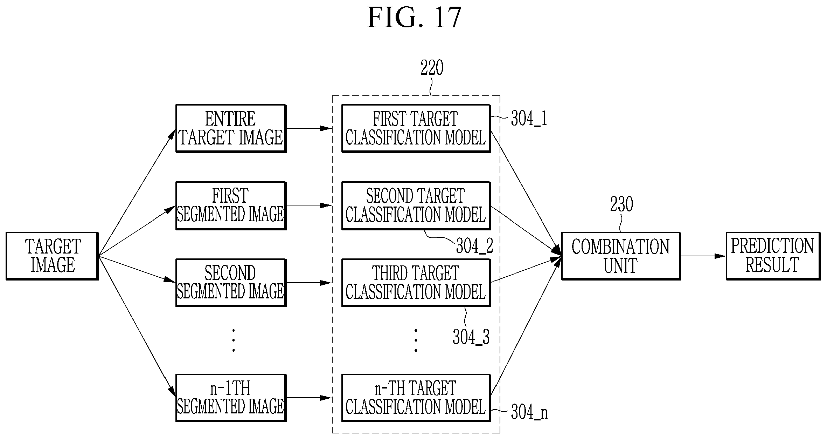

FIG. 17 is a diagram illustrating a combining process performed by the combination unit 230 according to the second exemplary embodiment of the present disclosure.

Referring to FIG. 17, according to the second exemplary embodiment of the present disclosure, the combination unit 230 may combine (synthesize) the plurality of bone disease classification results using the ensemble algorithm. For example, the combination unit 230 may average the logit or probability values, which are the bone disease classification result values inferred from each classification model, and compare the average value with a reference value to determine whether there is the bone disease (osteoporosis/non-osteoporosis). In this case, according to the exemplary embodiment of the present disclosure, when combining the plurality of bone disease classification results, the combination unit 230 may exclude one or more results and calculate the average value only for the remaining results to determine whether there is the bone disease (osteoporosis/non-osteoporosis). Meanwhile, according to an exemplary embodiment of the present disclosure, various techniques other than averaging may be applied as an ensemble technique. For example, one or a combination of voting, bagging, boosting, and stacking may be used as an ensemble technique.

In this way, according to the exemplary embodiment of the present disclosure, since the final classification results are derived by integrating the plurality of inference results inferred by applying the plurality of classification models (artificial intelligence models) to various bone areas included in the segmented image using the ensemble algorithm, it is possible to sufficiently reflect the characteristic information of various anatomical areas while effectively excluding specific areas that may cause a degradation in classification performance. Therefore, even if an artifact is included in the segmented images (especially, an image segmented by cropping), there is an advantage in that the performance degradation may be prevented without introducing a separate auxiliary model for artifact recognition.

In addition, in order to train the classification model with higher accuracy and infer the classification results, it is necessary to use not only the plurality of segmented images but also the entire image. The reason is as follows.

In the chest X-ray image (CXR image), anatomical locations within a subject may differ due to factors such as the skill of a photographer, equipment, and race. In the exemplary embodiment of the present disclosure, a separate artificial intelligence model that detects bone areas or performs segmentation is not used, but the original CXR image is divided into equal parts (cropped) and then the segmented image including each of the anatomical areas is used as the training image or the target image. In this way, when the plurality of classification models (artificial intelligence models) are used only for the segmented images that determine each individual piece without using the classification model that analyzes the entire image, there may be a possibility of performance degradation depending on the quality of the CXR image. Therefore, it is important to input the entire image as the training image or target image and to classify the results by ensembling the determination results for each individual anatomical area based on the determination results that consider the entire area in the CXR.

The following describes the experimental results with Comparative Example to demonstrate the effectiveness according to the second exemplary embodiment of the present disclosure.

### 1. Preparation of Dataset

57,589 anonymized post-anterior view chest X-ray images and label data were provided from Asan Medical Center, South Korea (IRB No. 2019-1226). Based on this, the dataset for training the classification model of the second exemplary embodiment of the present disclosure and Comparative Example (training data, verification data) and the dataset for internal performance evaluation (internal test) were prepared as shown in Table 2.

**(Table 2)**

| Training Data | | | Validation Data | | | Test Data | | |
|---|---|---|---|---|---|---|---|---|
| Normal | Osteopenia | Osteoporosis | Normal | Osteopenia | Osteoporosis | Normal | Osteopenia | Osteoporosis |
| 28,681 | 17,367 | 2,731 | 4,040 | 2,458 | 323 | 673 | 652 | 664 |

### 2. Generation of Classification Model

### 2.1. Implementation Example 1: Generation of model (ensemble application model of a plurality of individual classification models of entire image and a plurality of segmented images)_ Corresponding to second exemplary embodiment of the present disclosure

Based on the prepared training dataset and validation dataset in Table 2, the entire image of chest X-ray image (CXR image) (input image resolution: 1024 × 1024) and (segmented) images (input image resolution: spine image - 512 × 1024, cervical spine/left and right shoulders - 512 × 512) of each area of a left shoulder, a right shoulder, a cervical spine, and a spine extracted from the entire image were prepared.

Using the InceptionV3 architecture that is a neural network model, a source classification model for transfer learning was generated by being trained with images labeled "normal" and "osteoporosis" among the entire CXR images and the segmented images for each area.

Images corresponding to the "normal" and "osteopenia" among the entire image and the segmented images for each area were categorized as "non-osteoporosis" and labeled, and images corresponding to "osteoporosis" were labeled as "osteoporosis". The entire image/segmented images for each area and its label were transfer-learned to the source classification model to generate the target classification model (expert model) for the entire image and individual the segmented images for each area. Using the ensemble algorithm, a model was generated that may output the "final inference classification result" by excluding the "preliminary inference classification result" with the lowest probability (or logit) among the "preliminary inference classification results (labels and probability values)" of five target classification models trained by area (entire image, left shoulder, right shoulder, neck, and spine) and calculating the average.

### 2.2. Comparative Example 1: Generation of model (single classification model) _ Partial modification of second exemplary embodiment of the present disclosure

Based on the prepared training dataset and validation dataset in Table 2, the entire image of chest X-ray image (CXR image) (input image resolution: 1024 × 1024) and (segmented) images (input image resolution: spine image - 512 × 1024, cervical spine /left and right shoulders - 512 × 512) of each part of a left shoulder, a right shoulder, a cervical spine, and a spine extracted from the entire image were prepared.

Among the images (segmented images) by area, the images corresponding to "normal" and "osteopenia" were labeled as "non-osteoporosis", and the images corresponding to "osteoporosis" were labeled as "osteoporosis". The images (segmented images) and labels by area were trained based on the InceptionV3 architecture to generate four individual classification models specialized for each of the anatomical areas.

### 2.3 Implementation Example 2: Generation of model (ensemble application model of a plurality of individual classification models excluding classification model of entire image)

Based on the prepared training dataset and validation dataset in Table 2, the entire image of chest X-ray image (CXR image) (input image resolution: 1024 × 1024) and (segmented) images (input image resolution: spine image - 512 × 1024, cervical spine /left and right shoulders - 512 × 512) of each part of a left shoulder, a right shoulder, a cervical spine, and a spine extracted from the entire image were prepared.

Using the InceptionV3 architecture that is the neural network model, the source classification model for transfer learning was generated by being trained with images having the labels "normal" and "osteoporosis" among the extracted images (segmented images) by area.

Among the images (segmented images) by area, the images corresponding to "normal" and "osteopenia" were categorized as "non-osteoporosis" and labeled, and the images corresponding to "osteoporosis" were labeled as "osteoporosis", and the segmented images and their labels were transfer-learned to the source classification model to generate the target classification model for individual segmented images for each area. By applying the ensemble algorithm, a model was generated that may output the "final inference classification result" by averaging the "preliminary inference classification results (labels and probability values)" of the target classification models for two types of anatomical areas ("cervical spine -spine", "cervical spine -left shoulder", "cervical spine -right shoulder", "spine-left shoulder", "spine-right shoulder" or "left shoulder-right shoulder").

### 3. Experimental Results_Internal Performance Evaluation (Internal Test) Results of Implementation Examples 1 and 2 and Comparative Example 1

In order to compare the performance of the classification model (source classification model-target classification model) according to the second exemplary embodiment of the present disclosure and the single classification model for each of the anatomical areas, the sensitivity, specificity, positive predictive value, and negative predictive value were evaluated based on the classification accuracy (AUC) and true positive (TP), true negative (TN), false positive (FP), and false negative (FN).

As may be seen from Tables 3 and 4, the classification model according to Implementation Example 1 showed a high level of classification accuracy (AUC) compared to the classification model of Comparative Example 1, and a high level of negative predictive value (NPV) compared to Comparative Example 1. When a patient classified as negative for osteoporosis patient has actual disease, since the osteoporosis of the patient may deteriorate or the fracture may occur, it is noteworthy that that a high level of negative prediction value is achieved.

**(Table 3)**

| | Comparative Example1 | | | |
|---|---|---|---|---|
| | Left Scapula | Right Scapula | Cervical spine | Spine |
| Accuracy (AUC) | 0.8986 | 0.9077 | 0.9012 | 0.9092 |
| TP | 504 | 549 | 513 | 496 |
| TN | 1141 | 1081 | 1133 | 1148 |
| FP | 185 | 245 | 193 | 178 |
| FN | 159 | 114 | 150 | 167 |
| Sensitivity, % | 76.02 | 82.81 | 77.38 | 74.81 |
| Specificity, % | 86.05 | 81.52 | 85.44 | 86.58 |
| Positive Predictive Value (PPV), % | 73.15 | 69.14 | 72.66 | 73.59 |
| Negative Predictive Value (NPV), | 87.77 | 90.46 | 88.31 | 87.30 |

**(Table 4)**

| | Implementation Example 1 | Implementation Example 2 | | | | | |
|---|---|---|---|---|---|---|---|
| | Entire image+ partial image(cer vical spine +spine+left shoulder+ right shoulder) | Partial image 1 (cervical spine + spine) | Partial image 2 (cervical spine + left shoulder) | Partial image 3 (cervical spine + right shoulder) | Partial image 4 (spine + left shoulder) | Partial image 5 (spine + left shoulder) | Partial image 6 (left shoulder + right shoulder) |
| Accuracy (AUC) | 0.9388 | 0.9241 | 0.9166 | 0.92 | 0.9209 | 0.9255 | 0.9139 |
| TP | 576 | 483 | 491 | 520 | 467 | 500 | 501 |
| TN | 1146 | 1202 | 1193 | 1174 | 1207 | 1192 | 1155 |
| FP | 180 | 124 | 133 | 152 | 119 | 134 | 171 |
| FN | 87 | 180 | 172 | 143 | 196 | 163 | 162 |
| Sensitivity, % | 86.88 | 72.85 | 74.06 | 78.43 | 70.44 | 75.41 | 75.57 |
| Specificity, % | 86.43 | 90.65 | 89.97 | 88.54 | 91.03 | 89.89 | 87.10 |
| Positive Predictive Value (PPV), % | 76.19 | 79.57 | 78.69 | 77.38 | 79.69 | 78.86 | 74.55 |
| Negative Predictive Value (NPV), | 92.94 | 86.98 | 87.40 | 89.14 | 86.03 | 87.97 | 87.70 |

In addition, the classification model of Implementation Example 2 showed a high level of classification accuracy (AUC) compared to the classification model of Comparative Example 1, and a high level of negative predictive value (NPV) compared to Comparative Example 1. It is noteworthy that when a patient classified as negative for osteoporosis patient has actual disease, it may cause deterioration or fracture of osteoporosis, resulting in a high level of negative prediction.

However, the classification model of Implementation Example 2 (using only the segmented images, not the entire image) showed a slightly lower level of classification accuracy (AUC) and a slightly lower level of negative predictive value (NPV) compared to the classification model of Implementation Example 1.

According to the second exemplary embodiment of the present disclosure described above, since the target classification model is trained through the target training labels (non-osteoporosis/osteoporosis) divided into two categories, there is an advantage in that the target classification model may be sufficiently trained even with a relatively small number of images of the normal person, the osteopenia patient, and the osteoporosis patient.

Meanwhile, unlike the second exemplary embodiment of the present disclosure, when the sufficient training image data of the normal person, the osteopenia patient, and the osteoporosis patient are provided, the target classification model may be trained with the target training labels (normal/osteopenia/osteoporosis) divided into three more detailed categories, and more detailed bone diseases (normal/osteopenia/osteoporosis) may be classified for the target image using the trained target classification model.

Hereinafter, a classification model training method and a bone disease classification method according to a third exemplary embodiment of the present disclosure will be described.

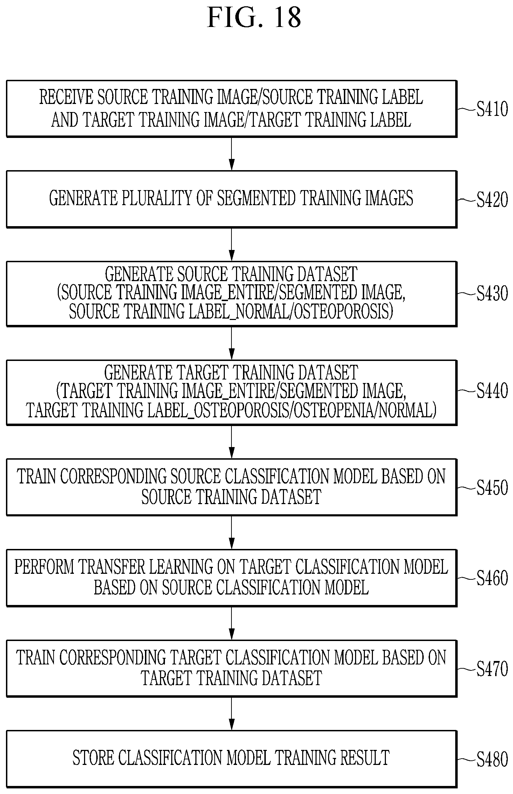

FIG. 18 is a diagram illustrating a classification model training method according to the third exemplary embodiment of the present disclosure. The classification model training method according to the third exemplary embodiment of the present disclosure is almost similar to the operation of the classification model training device 300 according to the second exemplary embodiment of the present disclosure described with reference to FIGS. 11 and 12. Accordingly, the classification model training method according to the third exemplary embodiment of the present disclosure will be described below with reference to the classification model training device 300 illustrated in FIGS. 11 and 12.

The classification model training device 300 receives the source training images/source training labels and the target training images/target training labels as the training data. (S410)

That is, the classification model training device 300 receives the source training images, which are the full CXR images of the normal person and the osteoporosis patient, and the source training labels (normal/osteoporosis), and receives the target training images, which are the full CXR images of the normal person, the osteopenia patient, and the osteoporosis patient, and the target training labels (normal/osteopenia/osteoporosis).

Thereafter, the image segmentation unit 310 of the classification model training device 300 segments the received source training image and target training image into the plurality of segmented images each corresponding to the anatomical areas. (S420)

The training dataset generation unit 320 generates the plurality of source training datasets based on the entire CXR image input as the source training images and the source training labels (normal/osteoporosis) related thereto, and the plurality of segmented images segmented by the image segmentation unit 310. (S430)

The training dataset generation unit 320 generates the plurality of target training datasets based on the entire CXR image input as a target training image and the target training labels (normal/osteopenia/osteoporosis) related thereto, and the plurality of segmented images segmented by the image segmentation unit 310. (S440)

The source classification model training unit 330 trains the corresponding source classification model by using the plurality of source training datasets composed of the training images of the normal person and the osteoporosis patient, which is relatively easy to train, and the source training labels (normal/osteoporosis), respectively, as the training data for the corresponding source classification model. (S450)

The transfer learning unit 350 performs the transfer learning on the target classification model based on the trained source classification model. (S460)

That is, the transfer learning unit 350 performs the transfer learning on the target classification model based on the artificial intelligence neural network parameters of the trained source classification model. (S460)

The target classification model training unit 340 uses each of the plurality of target training datasets composed of the target training images and target the training labels (normal/osteopenia/osteoporosis) of the normal person, the osteopenia patient, and the osteoporosis patient for the transfer-learned target classification model as the training data for the corresponding target classification model, and trains the corresponding target classification model. (S470)

The target classification model training unit 340 stores the learning parameters (weights, biases, etc.) which are the training results of the target classification model based on the target training dataset in memory or storage.

### (S480)

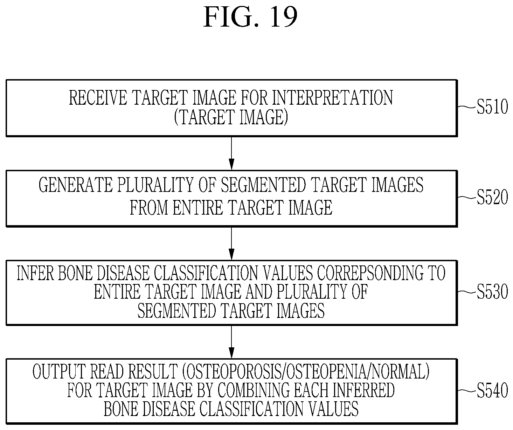

FIG. 19 is a diagram illustrating a bone disease classification method according to the third exemplary embodiment of the present disclosure. The bone disease classification method according to the third exemplary embodiment of the present disclosure is almost similar to the operation of the bone disease classification device according to the second exemplary embodiment of the present disclosure described with reference to FIGS. 11, 15, and 17. Accordingly, the bone disease classification method according to the third exemplary embodiment of the present disclosure will be described below with reference to the bone disease classification device 200 illustrated in FIGS. 11, 15, and 17.

The image segmentation unit 210 receives the CXR image to be read (hereinafter also referred to as the 'target image') (S510), and segments the entire target image into images corresponding to each of the anatomical areas. (S520)

In this case, the image segmentation unit 210 of the bone disease classification device 200 segments the entire image into the images corresponding to each of the anatomical areas using the same segmentation method as the image segmentation unit 210 of the classification model training device 300.

The bone disease classification inference unit 220 inputs the entire target image and the plurality of segmented images segmented by the image segmentation unit 210 into the corresponding classification model among the plurality of trained target classification model groups 304 performed by the classification model training device 300, and infers the bone disease classification results for each image (entire image or segmented image). (S530)

In this case, the classification results inferred by the bone disease classification inference unit 220 may be logit or probability values.

The combination unit 230 combines the plurality of bone disease classification results inferred by the bone disease classification inference unit 220 using the ensemble algorithm to diagnose whether there is the final bone disease (osteoporosis/osteopenia/normal). (S540)

The exemplary embodiments described above may be implemented in the form of a computer program that may be executed through various components on a computer, and such a computer program may be recorded on a computer-readable medium. In this case, the medium may include a magnetic medium such as a hard disk, a floppy disk, and a magnetic tape, an optical recording medium such as a CD-ROM and a DVD, a magneto-optical medium such as a floptical disk, and a hardware device specifically configured to store and execute program instructions, such as a ROM, a RAM, and a flash memory.

Unless there is a description that clearly describes or contradicts the order of steps constituting the method according to the exemplary embodiment of the present disclosure, the steps may be performed in an appropriate order, and the present disclosure is not limited according to the order of the description of the steps. All examples or exemplary terms used in the present disclosure are merely for the purpose of describing the present disclosure in detail, and the scope of the present disclosure is not limited thereto. In addition, those skilled in the art may recognize that various modifications, combinations, and changes may be made within the scope of the patent claims or their equivalents.

Although the embodiments of the present disclosure have been described in detail above, the scope of the present disclosure is not limited thereto, and various modifications and improvements by those of ordinary skill in the field to which the present disclosure pertains belong to the scope of the present disclosure.

### <Description of symbols>

10: System for diagnosing osteoporosis, 20: Medical image storage device, 30: User terminal, 11: Processor, 13: Memory, 15: Storage, 17: Communication interface, 19: Bus, 50: Neural network, 51: Input layer, 52: Hidden layer, 53: Output layer, 100: Classification model training device, 110: Image segmentation unit, 120: Training dataset generation unit, 130: Classification model training unit, 200: Bone disease classification prediction device, 210: Image segmentation unit, 220: Bone disease classification inference unit, 230: Combination unit, 300: Classification model training device, 303: Source classification model group, 304: Target classification model group, 130: Image segmentation unit, 320: Training dataset generation unit, 330: Source classification model training unit, 340: Target classification model training unit, 350: Transfer learning unit

## Claims

1. A system for diagnosing osteoporosis, comprising:
a classification model training device that includes a plurality of classification models, one or more of the plurality of classification models being artificial intelligence models corresponding to each of the plurality of segmented images which are obtained by segmenting an entire image corresponding to each anatomical area, and trains a corresponding classification model based on the plurality of segmented images and training labels corresponding to each segmented image, the training label being labeled as normal or the osteoporosis; and
a bone disease classification device that diagnoses whether there is the osteoporosis by inputting the plurality of segmented images segmented from a target image for interpretation to the corresponding classification model of the classification model training device.

2. The system of claim 1, wherein:
the plurality of classification models includes a first classification model corresponding to the entire image, and
the classification model training device trains the first classification model based on the entire image and the corresponding training label.

3. The system of claim 2, wherein:
the entire image is a chest X-ray image.

4. The system of claim 2, wherein:
the classification model training device includes:
a first image segmentation unit that segments the entire image into the anatomical areas to generate the segmented image;
a training dataset generation unit that generates a plurality of training datasets based on an entire image of a normal person or an osteoporosis patient input from the outside and a plurality of segmented images segmented by the first image segmentation unit, each training dataset including a training image which is the entire image or the segmented image, and the training label labeled as the normal or the osteoporosis; and
a plurality of classification model training units that train each of the corresponding classification models based on the plurality of training datasets generated by the training dataset generation unit.

5. The system of claim 4, wherein:
the first image segmentation unit crops and segments the entire image into images corresponding to each anatomical area, and
the training image generated by the training dataset generation unit includes an entire chest image and one or more segmented images of right clavicle-scapula, left clavicle-scapula, cervical spine, and thoracic/lumbar spine.

6. The system of any one of claim 1 to claim 5, wherein:
the bone disease classification device includes:
a second image segmentation unit that segments the target image for interpretation into the anatomical areas to generate the plurality of segmented images; and
a bone disease classification inference unit that inputs the entire target image for interpretation and the plurality of segmented images segmented by the second image segmentation unit to the corresponding classification model among the plurality of classification models of the classification model training device, and infers bone disease classification results for each input image.

7. The system of claim 6, wherein:
the bone disease classification device further includes:
a combination unit that combines a plurality of bone disease classification results inferred by the bone disease classification inference unit using an ensemble algorithm to diagnose whether the target image for interpretation corresponds to an osteoporosis image or a normal person image.

8. A system for diagnosing osteoporosis, comprising:
a classification model training device that performs primary training on a plurality of source classification models based on first training images including segmented images segmented from entire images of a normal person and an osteoporosis patient, transfers a primary training result, and performs secondary training on a plurality of target classification model s based on secondary training images including segmented images segmented from an entire images of the normal person, an osteopenia patient, and the osteoporosis patient; and
a bone disease classification device that diagnoses whether there is the osteoporosis by inputting a plurality of segmented images segmented from a target image for interpretation to each of the corresponding target classification models.

9. The system of claim 8, wherein:
the plurality of source classification models and the plurality of target classification models each include a first source classification model and a first target classification model corresponding to the entire image.

10. The system of claim 9, wherein:
the entire image is a chest X-ray image.

11. The system of claim 9, wherein:
the classification model training device includes:
a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area;
a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as non-osteoporosis or the osteoporosis;
a plurality of source classification model training units that perform the primary training on the corresponding source classification model based on the plurality of source training datasets; and
a plurality of target classification model training units that performs the secondary training on the corresponding target classification model based on the plurality of target training datasets.

12. The system of claim 9, wherein:
the classification model training device includes:
a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area;
a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as normal, osteopenia, or the osteoporosis;
a plurality of source classification model training units that perform the primary training on the corresponding source classification model based on the plurality of source training datasets; and
a plurality of target classification model training units that performs the secondary training on the corresponding target classification model based on the plurality of target training datasets.

13. The system of claim 11 or claim 12, wherein:
the classification model training device further includes a transfer learning unit that performs transfer learning on a corresponding target classification model training unit based on hidden layer parameters which are training results of the source classification model training unit.

14. The system of claim 13, wherein:
the first image segmentation unit crops and segments the entire image into images corresponding to each anatomical area, and
the training image generated by the training dataset generation unit includes an entire chest image and one or more segmented images of right clavicle-scapula, left clavicle-scapula, cervical spine, and thoracic/lumbar spine.

15. The system of any one of claim 8 to claim 12, wherein:
the bone disease classification device includes:
a second image segmentation unit that segments the target image for interpretation into the anatomical areas to generate the plurality of segmented images; and
a bone disease classification inference unit that inputs the entire target image for interpretation and the plurality of segmented images segmented by the second image segmentation unit to the corresponding classification model among the plurality of target classification models, and infers bone disease classification results for each input image.

16. The system of claim 15, wherein:
the bone disease classification device further includes a combination unit that combines a plurality of bone disease classification results inferred by the bone disease classification inference unit using an ensemble algorithm to diagnose whether the target image for interpretation corresponds to an osteoporosis image.

17. The system of claim 16, wherein:
the bone disease classification device diagnoses the target image for interpretation as an osteoporosis image or a non-osteoporosis image.

18. The system of claim 16, wherein:
the bone disease classification device diagnoses the target image for interpretation as an osteoporosis image, an osteopenia image, or a normal person's image.

19. A bone disease classification device that diagnoses whether there is the osteoporosis by accessing a classification model training device that includes a plurality of classification models corresponding to an entire image or each of a plurality of segmented images which are obtained by segmenting the entire image corresponding to each anatomical area, wherein
the bone disease classification device inputs a target image for interpretation or a plurality of segmented images segmented from the target image for interpretation to a corresponding classification model among the plurality of classification models to diagnose whether the target image for interpretation is the osteoporosis.

20. The bone disease classification device of claim 19, further comprising:
an image segmentation unit that segments the target image for interpretation into the anatomical areas to generate the plurality of segmented images; and
a bone disease classification inference unit that inputs the entire target image for interpretation and the plurality of segmented images segmented by the image segmentation unit to the corresponding classification model among the plurality of classification models, and infers bone disease classification results for each input image.

21. The bone disease classification device of claim 20, further comprising:
a combination unit that combines a plurality of bone disease classification results inferred by the bone disease classification inference unit using an ensemble algorithm to diagnose whether the target image for interpretation corresponds to an osteoporosis image.

22. The bone disease classification device of claim 21, wherein:
the bone disease classification device diagnoses the target image for interpretation as the osteoporosis image or a non-osteoporosis image.

23. The bone disease classification device of claim 21, wherein:
the bone disease classification device diagnoses the target image for interpretation as the osteoporosis image, an osteopenia image, or a normal person image.

24. The bone disease classification device of any one of claim 19 to claim 23, wherein:
the plurality of classification training models are classification training models that are transferred from training results of a first classification model that is primarily trained with training data of a normal person and an osteoporosis patient and is secondarily trained with training data of the normal person, an osteopenia patient, and the osteoporosis patient.

25. A classification model training device, wherein
the classification model training device performs primary training on a plurality of source classification models based on first training images including segmented images segmented from entire images of a normal person and an osteoporosis patient, transfers the primary training result, and performs secondary training on a plurality of target classification models based on secondary training images including segmented images segmented from an entire image of the normal person, an osteopenia patient, and the osteoporosis patient.

26. The classification model training device of claim 25, wherein:
the plurality of source classification models and the plurality of target classification models each include a first source classification model and a first target classification model corresponding to the entire image.

27. The classification model training device of claim 26, comprising:
a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area;
a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as a non-osteoporosis or the osteoporosis;
a plurality of source classification model training units that performs the primary training on the corresponding source classification model based on the plurality of source training datasets; and
a plurality of target classification model training units that performs the secondary training on the corresponding target classification model based on the plurality of target training datasets.

28. The classification model training device of claim 26, comprising:
a first image segmentation unit that receives a source training image which is the entire image of the normal person or the osteoporosis patient, segments the source training image into images corresponding to each anatomical area, receives a target training image which is the entire image of the normal person, the osteopenia patient, or the osteoporosis patient, and segments the target training image into images corresponding to each anatomical area;
a training dataset generation unit that generates a plurality of source training datasets based on the source training image and a plurality of segmented images segmented from the source training image, each source training dataset including the first training image which is an image segmented from the source training image or the source training image, and a source training label labeled as normal or osteoporosis, and a plurality of target training datasets based on the target training image and a plurality of segmented images segmented from the target training image, each target training dataset including the second training image which is an image segmented from the target training image or the target image, and a target training label labeled as the normal, a osteopenia, or the osteoporosis;
a plurality of source classification model training units that performs the primary training on the corresponding source classification model based on the plurality of source training datasets; and
a plurality of target classification model training units that performs the secondary training on the corresponding target classification model based on the plurality of target training datasets.

29. The classification model training device of claim 27 or claim 28, further comprising:
a transfer learning unit that performs transfer learning on a corresponding target classification model training unit based on hidden layer parameters which are training results of the source classification model training unit.

30. A method for diagnosing osteoporosis that diagnoses whether there is the osteoporosis from a target image for interpretation, comprising
segmenting the target image for interpretation into a plurality of images corresponding to each anatomical area;
inputting the target image for interpretation and the plurality of segmented images to a corresponding classification model among a plurality of classification models to infer bone disease classification results for each input image, one or more of the plurality of classification models being artificial intelligence models corresponding to each of the plurality of segmented images which are obtained by segmenting the entire image corresponding to each anatomical area; and
diagnosing whether the target image for interpretation is the osteoporosis by combining the plurality of inferred bone disease classification results using an ensemble algorithm.

31. The method of claim 30, further comprising:
training the plurality of classification models based on the entire image of a normal person or an osteoporosis patient,
wherein the training of the classification models includes:
segmenting an entire image into the anatomical areas to generate the segmented image;
generating a plurality of training datasets based on the entire training image and the plurality of segmented images, each training dataset including a training image that is the entire image or the segmented image and a training label labeled as normal or the osteoporosis; and
training corresponding classification models, respectively, based on the plurality of training datasets.

32. The method of claim 31, wherein:
the entire image is a chest X-ray image, and
the entire image is cropped into images corresponding to each anatomical area to generate the plurality of segmented images.

33. The method of claim 30, further comprising:
training the plurality of classification models based on an entire image of a normal person, a osteopenia patient, or an osteoporosis patient,
wherein the plurality of classification training models are a plurality of target classification training models that are transferred from training results of a plurality of source classification models that are primarily trained with a first training images of the normal person and the osteoporosis patient, and are classification training models that are secondarily trained with a second training image of a normal person, an osteopenia patient, and the osteoporosis patient.

34. The method of claim 33, further comprising:
segmenting a source training image, which is an entire image of the normal person or the osteoporosis patient, into images corresponding to each anatomical area;
generating a plurality of source training datasets based on the source training image and a plurality of segmented images segmented from the source training image, each of the source training datasets including the first training image that is an image segmented from the source training image or the source training image, and a source training label labeled as the normal or the osteoporosis;
performing the primary training on the corresponding source classification model based on the plurality of source training datasets;
performing transfer learning on the corresponding target classification model based on hidden layer parameters that are training results of the source classification model;
segmenting a target training image, which is the entire image of the normal person, the osteopenia patient, or the osteoporosis patient, into images corresponding to each anatomical area;
generating a plurality of target training datasets based on the target training image and a plurality of segmented images segmented from the target training image, each of the target training datasets including the second training image that is an image segmented from the target training image or the target training image, and a target training label labeled as the non-osteoporosis or the osteoporosis; and
performing the secondary training on the corresponding target classification model based on the plurality of target training datasets.

35. The method of claim 33, further comprising:
segmenting a source training image, which is an entire image of the normal person or the osteoporosis patient, into images corresponding to each anatomical area;
generating a plurality of source training datasets based on the source training image and a plurality of segmented images segmented from the source training image, each of the source training datasets including the first training image that is an image segmented from the source training image or the source training image, and a source training label labeled as the normal or the osteoporosis;
performing the primary training on the corresponding source classification model based on the plurality of source training datasets;
performing transfer learning on the corresponding target classification model based on hidden layer parameters that are training results of the source classification model;
segmenting a target training image, which is the entire image of the normal person, the osteopenia patient, or the osteoporosis patient, into images corresponding to each anatomical area;
generating a plurality of target training datasets based on the target training image and a plurality of segmented images segmented from the target training image, each of the target training datasets including the second training image that is an image segmented from the target training image or the target training image, and a target training label labeled as the normal, the osteopenia, the osteoporosis; and
performing the secondary training on the corresponding target classification model based on the plurality of target training datasets.

36. A method for training a plurality of classification models based on an entire image of a normal person, an osteopenia patient, or an osteoporosis patient, comprising:
performing primary training on a plurality of source classification models based on first training images including segmented images segmented from entire images of a normal person and an osteoporosis patient; and
transferring primary training results and performing secondary training on a plurality of target classification models based on second training images including segmented images segmented from entire images of a normal person, an osteopenia patient, and an osteoporosis patient.

37. The method of claim 36, wherein:
the performing of the primary training includes:
segmenting a source training image, which is an entire image of the normal person or the osteoporosis patient, into images corresponding to each anatomical area;
generating a plurality of source training datasets based on the source training image and a plurality of segmented images segmented from the source training image, each of the source training datasets including the first training image that is an image segmented from the source training image or the source training image, and a source training label labeled as the normal or the osteoporosis; and
performing the primary training on the corresponding source classification model based on the plurality of source training datasets.

38. The method of claim 37, wherein:
the performing of the secondary training includes:
performing transfer learning on the corresponding target classification model based on hidden layer parameters that are training results of the source classification model;
segmenting a target training image, which is the entire image of the normal person, the osteopenia patient, or the osteoporosis patient, into images corresponding to each anatomical area;
generating a plurality of target training datasets based on the target training image and a plurality of segmented images segmented from the target training image, each of the target training datasets including the second training image that is an image segmented from the target training image or the target training image, and a target training label labeled as the non-osteoporosis or the osteoporosis; and
performing the secondary training on the corresponding target classification model based on the plurality of target training datasets.

39. The method of claim 37, wherein:
the performing of the secondary training includes:
performing transfer learning on the corresponding target classification model based on hidden layer parameters that are training results of the source classification model;
segmenting a target training image, which is an entire image of the normal person, the osteopenia patient, or the osteoporosis patient, into images corresponding to each anatomical area;
generating a plurality of target training datasets based on the target training image and a plurality of segmented images segmented from the target training image, each of the target training datasets including the second training image that is an image segmented from the target training image or the target training image, and a target training label labeled as the normal, the osteopenia, the osteoporosis; and
performing the secondary training on the corresponding target classification model based on the plurality of target training datasets.

40. A computer, comprising:
at least one processor implemented to execute a computer-readable command,
wherein the at least one processor
segments a target image for interpretation into a plurality of images corresponding to each anatomical area,
inputs the target image for interpretation and the plurality of segmented images to a corresponding classification model among a plurality of classification models to infer bone disease classification results for each input image, one or more of the plurality of classification models being artificial intelligence models corresponding to each of the plurality of segmented images which are obtained by segmenting the entire image corresponding to each anatomical area, and
diagnoses whether the target image for interpretation is the osteoporosis by combining the plurality of inferred bone disease classification results using an ensemble algorithm.
